(19)

**Europäisches Patentamt**
**European Patent Office**
**Office européen des brevets**

(11)  **EP 4 165 658 B1**

(12)  **EUROPEAN PATENT SPECIFICATION**

(45) Date of publication and mention
of the grant of the patent:
**01.01.2025  Bulletin 2025/01**

(21) Application number: **21743320.0**

(22) Date of filing: **10.06.2021**

(51) International Patent Classification (IPC):
**G16H 50/20** *(2018.01)*    **G16H 40/63** *(2018.01)*
**G16H 40/67** *(2018.01)*    **G16H 50/30** *(2018.01)*

(52) Cooperative Patent Classification (CPC):
**G16H 20/30; G16H 40/63; G16H 40/67;**
**G16H 50/20; G16H 50/30; G16H 50/70**

(86) International application number:
**PCT/US2021/036817**

(87) International publication number:
**WO 2021/252768 (16.12.2021 Gazette 2021/50)**

(54) **WEARABLE INFECTION MONITOR**

TRAGBARER INFEKTIONSMONITOR

DISPOSITIF DE SURVEILLANCE D'INFECTION POUVANT ÊTRE PORTÉ SUR SOI

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **10.06.2020   US 202063037499 P**

(43) Date of publication of application:
**19.04.2023   Bulletin 2023/16**

(73) Proprietor: **Whoop, Inc.**
**Boston, MA 02215 (US)**

(72) Inventors:
• **CAPODILUPO, John Vincenzo**
**Boston, MA 02215 (US)**
• **CAPODILUPO, Emily Rachel**
**Boston, MA 02215 (US)**
• **LEE, Victoria Harrison**
**Boston, MA 02215 (US)**

(74) Representative: **Black, Diego**
**Withers & Rogers LLP**
**2 London Bridge**
**London SE1 9RA (GB)**

(56) References cited:
**EP-A1- 3 479 758       WO-A2-2017/032873
US-A1- 2019 167 209    US-A1- 2019 209 022**

• **CAPODILUPO EMILY: "The Importance of Respiratory Rate Tracking During The COVID-19 Pandemic"**, 1 April 2020 (2020-04-01), XP093105900, Retrieved from the Internet <URL:https://www.whoop.com/us/en/thelocker/respiratory-rate-tracking-coronavirus/> [retrieved on 20231127]
• **WHOOP: "WHOOP Investigating Respiratory Rate Pattern and Relationship with COVID-19 Symptoms"**, 1 April 2020 (2020-04-01), XP093098145, Retrieved from the Internet <URL:https://www.prnewswire.com/news-releases/whoop-investigating-respiratory-rate-pattern-and-relationship-with-covid-19-symptoms-301033339.html> [retrieved on 20231105]
• **ETHERINGTON DARRELL: "Researchers to study if startup's wrist-worn wearable can detect early COVID-19 respiratory issues | TechCrunch"**, 1 April 2020 (2020-04-01), XP093098146, Retrieved from the Internet <URL:https://techcrunch.com/2020/04/01/researchers-to-study-if-startups-wrist-worn-wearable-can-detect-early-covid-19-respiratory-issues/> [retrieved on 20231105]
• **STOJANOVIC RADOVAN ET AL: "A Headset Like Wearable Device to Track COVID-19 Symptoms"**, 2020 9TH MEDITERRANEAN CONFERENCE ON EMBEDDED COMPUTING (MECO), IEEE, 8 June 2020 (2020-06-08), pages 1 - 4, XP033788349, DOI: 10.1109/MECO49872.2020.9134211

EP 4 165 658 B1

- KRANCK GUSTAF: "The Vagus ECG smartwatch to monitor changes in cardiac-respiratory synchronization and QRST amplitude during the progress of Cardiac-Respiratory infection.", 5 March 2020 (2020-03-05), XP055839257, Retrieved from the Internet <URL:https://vagus.co/wp-content/uploads/2020/03/Open-Study-with-Vagus-ECG-smartwatch-for-Coronavirus-progress-monitoring.pdf> [retrieved on 20210908]

## Description

CROSS-REFERENCE TO RELATED APPLICATIONS

This application claims priority to U.S. Provisional Application No. 63/037,499 filed on June 10, 2020.

TECHNICAL FIELD

The present disclosure generally relates to monitoring and/or detecting a presence of a contagious disease and/or other condition that manifests variations in baseline physiological signals based on information received from a wearable physiological monitor.

BACKGROUND

Wearable physiological monitors can provide a wealth of continuous physiological data from a wearer. While such devices have been used for general fitness, there remains a need for physiological monitors that support early detection and intervention for contagious diseases or other conditions that manifest variations in baseline physiological signals.

Background information can be found in the set of internet disclosures regarding the wearable infection monitor Whoop, such as Capodilupo Emily: "The Importance of Respiratory Rate Tracking During The COVID-19 Pandemic", 1 April 2020 (2020-04-01), XP093105900, www.whoop.com/us/en/thelocker/respiratory-rate-tracking-coronavirus/, and also from

- US 2019/167209 A1 is directed to non-invasive monitoring devices to acquire patient condition signals and a respiratory distress monitoring circuit to monitor a state of the respiratory distress using the patient condition signals.
- EP 3479758 A1 is directed to a system and method for breathing pattern extraction from ppg signals, where, using the filter comprising the plurality of filter coefficients, a filtered PPG signal is generated by removing DC component from PPG signals obtained from a wearable device being worn by a subject. The filtered PPG signal is indicative of the breathing pattern of the subject.
- WO 2017/032873 A2 is directed to systems and methods for monitoring and management of chronic disease, where a management device may be configured to analyze the physiological and/or environmental parameters to detect a trigger pattern of the parameters, the trigger pattern indicative of a probable event of exacerbation of the chronic respiratory and/or cardiac condition.
- STOJANOVIC RADOVAN ET AL: "A Headset Like Wearable Device to Track COVID-19 Symptoms" which is directed to a headset with a built-in microphone to detect breathing problems, respiration rate and cough and a heart-rate sensor in the form of PPG ear-clip.

SUMMARY

The invention is defined by the appended claims. Heart rate data from a wearable physiological monitor can be used to determine a respiratory rate for a wearer. Using this respiratory rate data, a respiratory rate baseline for a wearer can be determined and used to detect variations from the baseline that indicate onset of conditions such as Covid-19 or other respiratory infections and the like.

In one aspect, a computer program product disclosed herein includes computer executable code embodied in a non-transitory computer readable medium that, when executing on one or more computing devices, performs the steps of: acquiring heart rate data from a user with a wearable physiological monitor; determining a historical respiratory rate pattern for the user at a first number of predetermined daily intervals during periods of deep sleep by the user based on the heart rate data, the historical respiratory rate pattern characterizing one or more features of a respiratory activity of the user during the first number of predetermined daily intervals during periods of deep sleep by the user; determining a current respiratory rate pattern for the user during a second predetermined daily interval based on the heart rate data during a second period of deep sleep; evaluating the one or more features of the current respiratory rate pattern; comparing the one or more features of the current respiratory rate pattern to the one or more features of the historical respiratory rate pattern; and, in response to a predetermined difference between the one or more features of the current respiratory rate pattern and the one or more features of the historical respiratory rate pattern, creating an indicator of a likelihood of a respiratory infection of the user.

Implementations may include one or more of the following features. The first number of predetermined daily intervals and the second predetermined daily interval may include sleep intervals detected using data using the wearable physiological monitor. Comparing one or more features may be performed on a remote server. The indicator may be transmitted from the remote server to a device associated with the user. The device may be the wearable physiological monitor. The device may be at least one of a laptop computer, a tablet, or a cellular phone associated with the user.

Implementations may include acquiring a physiological data signal from a user of a wearable device over a period of time including a recent window and at least one historical window preceding the recent window, and automatically generating an indicator for likelihood of an infection of the user at least once per day based on a comparison of one or more features of the physiological data signal during the recent window to the one or more features of the physiological data signal during the at least one historical window.

Implementations may include transmitting the physiological data signal to a server, automatically generating the indicator at the server, and transmitting the indicator to a device associated with the user for display. Implementations may further include automatically generating the indicator on the wearable device and transmitting the indicator to a device associated with the user. The indicator for likelihood of the infection of the user may be an indicator for a likelihood of a respiratory infection of the user. The infection may be a Covid-19 infection. The recent window may be a sleep interval for the user detected by the wearable device. The historical window may include one or more prior sleep intervals for the user detected by the wearable device. The historical window may include a number of intervals sufficient to establish a pre-infection baseline for a health respiratory pattern. The physiological data signal may include heart rate data for the user. The physiological data signal may provide a proxy for a respiratory pattern of the user. Implementations may further include training a machine classifier to return a probability that a set of values for the one or more features is indicative of the infection, and applying the machine classifier to the one or more features of the physiological data signal during the recent window.

In one aspect, a system disclosed herein includes a server including one or more processors configured to receive from a user with a wearable physiological monitor heart rate data and to evaluate a respiratory health of a user by performing the steps of: determining a historical respiratory rate pattern for the user at a first number of predetermined daily intervals based on the heart rate data during deep sleep, the historical respiratory rate pattern characterizing one or more features of a typical respiratory rate pattern during the one or more predetermined daily intervals during deep sleep; determining a current respiratory rate pattern for the user during a second predetermined daily interval based on the heart rate data during a period of deep sleep; evaluating the one or more features of the current respiratory rate pattern; comparing the one or more features of the current respiratory rate pattern to the one or more features of the typical respiratory rate pattern; and, in response to a predetermined difference between the one or more features of the current respiratory rate pattern and the one or more features of the typical respiratory rate pattern, creating an indicator of a likelihood of a respiratory infection of the user.

Implementations may include one or more of the following features. The system may further include a wearable physiological monitor configured to continuously acquire heart rate data from the user and transmit the heart rate data to the server. The system may further include a user device configured to receive an alert from the server and display the alert to the user when the likelihood of the respiratory infection is above a predetermined threshold.

BRIEF DESCRIPTION OF THE DRAWINGS

The foregoing and other objects, features, and advantages of the devices, systems, and methods described herein will be apparent from the following description of particular embodiments thereof, as illustrated in the accompanying drawings. The drawings are not necessarily to scale, emphasis instead being placed upon illustrating the principles of the devices, systems, and methods described herein. In the drawings, like reference numerals generally identify corresponding elements.

Fig. 1 illustrates front and back perspective views of a wearable system configured as a bracelet including one or more straps.

Fig. 2 illustrates a wearable physiological measurement system.

Fig. 3 illustrates placement of a wearable physiological measurement system on a user's wrist.

Fig. 4 illustrates a side view of a physiological measurement system including a strap that is not coupled to a modular head portion.

Fig. 5 illustrates a side view of a physiological measurement system in which a modular head portion is removably coupled to the strap.

Fig. 6 is a flow chart illustrating a signal processing algorithm for generating a sequence of heart rates for every detected heartbeat that may be embodied in computer-executable instructions stored on one or more non-transitory computer-readable media.

Fig. 7 is a flow chart illustrating a method of determining an intensity score.

Fig. 8 is a flow chart illustrating a method by which a user may use intensity and recovery scores.

Fig. 9 illustrates a display of an intensity score index indicated in a circular graphic component with an exemplary current score of 19.0 indicated.

Fig. 10 illustrates a display of a recovery score index indicated in a circular graphic component with a first threshold of 66% and a second threshold of 33% indicated.

Fig. 11A illustrates a recovery score graphic component with a recovery score and qualitative information corresponding to the recovery score.

Fig. 11B illustrates a recovery score graphic component with a recovery score and qualitative information corresponding to the recovery score.

Fig. 11C illustrates a recovery score graphic component with a recovery score and qualitative information corresponding to the recovery score.

Fig. 12 is a block diagram of a computing device that may be used herein.

Fig. 13 is a block diagram of a distributed computer system in which various aspects and functions in accord with the present disclosure may be practiced.

Fig. 14 is a diagram of a network environment suitable for a distributed implementation of embodiments described herein.

Fig. 15 is a flow chart illustrating a method for selecting modes of acquiring heart rate data.

Fig. 16 is a flow chart of a method for assessing recovery and making exercise recommendations.

Fig. 17 is a flow chart illustrating a method for detecting heart rate variability in sleep states.

Fig. 18 is a flow chart of a method for creating an indicator of a condition.

Fig. 19 is a flow chart of a method of infection monitoring.

Fig. 20 illustrates a physiological monitoring system.

DESCRIPTION

The embodiments will now be described more fully hereinafter with reference to the accompanying figures, in which preferred embodiments are shown.

Recitation of ranges of values herein are not intended to be limiting, referring instead individually to any and all values falling within the range, unless otherwise indicated herein, and each separate value within such a range is incorporated into the specification as if it were individually recited herein. The words "about," "approximately" or the like, when accompanying a numerical value, are to be construed as indicating a deviation as would be appreciated by one of ordinary skill in the art to operate satisfactorily for an intended purpose. Similarly, words of approximation such as "approximately" or "substantially" when used in reference to physical characteristics, should be understood to contemplate a range of deviations that would be appreciated by one of ordinary skill in the art to operate satisfactorily for a corresponding use, function, purpose, or the like. Ranges of values and/or numeric values are provided herein as examples only, and do not constitute a limitation on the scope of the described embodiments. Where ranges of values are provided, they are also intended to include each value within the range as if set forth individually, unless expressly stated to the contrary. The use of any and all examples, or exemplary language ("e.g.," "such as," or the like) provided herein, is intended merely to better describe the embodiments and does not pose a limitation on the scope of the embodiments. No language in the specification should be construed as indicating any unclaimed element as essential to the practice of the embodiments.

Exemplary embodiments provide physiological measurement systems, devices and methods for continuous health and fitness monitoring, and provide improvements to overcome the drawbacks of conventional heart rate monitors. One aspect of the present disclosure is directed to providing a lightweight wearable system with a strap that collects various physiological data or signals from a wearer. The strap may be used to position the system on an appendage or extremity of a user, for example, wrist, ankle, and the like. Exemplary systems are wearable and enable real-time and continuous monitoring of heart rate without the need for a chest strap or other bulky equipment which could otherwise cause discomfort and prevent continuous wearing and use. The system may determine the user's heart rate without the use of electrocardiography and without the need for a chest strap. Exemplary systems can thereby be used in not only assessing general well-being but also in continuous monitoring of fitness. Exemplary systems also enable monitoring of one or more physiological parameters in addition to heart rate including, but not limited to, body temperature, heart rate variability, motion, sleep, stress, fitness level, recovery level, effect of a workout routine on health and fitness, caloric expenditure, and the like.

A health or fitness monitor that includes bulky components may hinder continuous wear. Existing fitness monitors often include the functionality of a watch, thereby making the health or fitness monitor quite bulky and inconvenient for continuous wear. Accordingly, one aspect is directed to providing a wearable health or fitness system that does not include bulky components, thereby making the bracelet slimmer, unobtrusive and appropriate for continuous wear. The ability to continuously wear the bracelet further allows continuous collection of physiological data, as well as continuous and more reliable health or fitness monitoring. For example, embodiments of the bracelet disclosed herein allow users to monitor data at all times, not just during a fitness session. In some embodiments, the wearable system may or may not include a display screen for displaying heart rate and other information. In other embodiments, the wearable system may include one or more light emitting diodes (LEDs) to provide feedback to a user and display heart rate selectively. In some embodiments, the wearable system may include a removable or releasable modular head that may provide additional features and may display additional information. Such a modular head can be releasably installed on the wearable system when additional information display is desired and removed to improve the comfort and appearance of the wearable system. In other embodiments, the head may be integrally formed in the wearable system.

Exemplary embodiments also include computer-executable instructions that, when executed, enable automatic interpretation of one or more physiological parameters to assess the cardiovascular intensity experienced by a user (embodied in an intensity score or indicator) and the user's recovery after physical exertion or daily stress given sleep and other forms of rest (embodied in a recovery score). These indicators or scores may be stored and displayed in a meaningful format to assist a user in managing his health and exercise regimen. Exemplary computer-executable instructions may be provided in a cloud implementation.

Exemplary embodiments also provide a vibrant and interactive online community, in the form of a website, for displaying and sharing physiological data among users. A user of the website may include an individual whose health or fitness is being monitored, such as an individual wearing a wearable system disclosed herein, an athlete, a sports team member, a personal trainer or a coach. In some embodiments, a user may pick his/her own trainer from a list to comment on their performance. Exemplary systems have the ability to stream all physiological information wirelessly, directly or through a mobile communication device application, to an online website using data transfer to a cell phone/computer. This information, as well as any data described herein, may be encrypted (e.g., the data may include encrypted biometric data). Thus, the encrypted data may be streamed to a secure server for processing. In this manner, only authorized users will be able to view the data and any associated scores. In addition, or in the alternative, the website may allow users to monitor their own fitness results, share information with their teammates and coaches, compete with other users, and win status. Both the wearable system and the website allow a user to provide feedback regarding his/her day, exercise and/or sleep, which enables recovery and performance ratings.

In an exemplary technique of data transmission, data collected by a wearable system may be transmitted directly to a cloud-based data storage, from which data may be downloaded for display and analysis on a website. In another exemplary technique of data transmission, data collected by a wearable system may be transmitted via a mobile communication device application to a cloud-based data storage, from which data may be downloaded for display and analysis on a website.

In some embodiments, the website may be a social networking site. In some embodiments, the website may be displayed using a mobile website or a mobile application. In some embodiments, the website may be configured to communicate data to other websites or applications. In some embodiments, the website may be configured to provide an interactive user interface. The website may be configured to display results based on analysis on physiological data received from one or more devices. The website may be configured to provide competitive ways to compare one user to another, and ultimately a more interactive experience for the user. For example, in some embodiments, instead of merely comparing a user's physiological data and performance relative to that user's past performances, the user may be allowed to compete with other users and the user's performance may be compared to that of other users.

Certain terms are defined below to facilitate understanding of exemplary embodiments.

The term "user" as used herein, refers to any type of animal, human or non-human, whose physiological information may be monitored using an exemplary wearable physiological monitoring system.

The term "body," as used herein, refers to the body of a user.

The term "continuous," as used herein in connection with heart rate data collection, refers to collection of heart rate data at a sufficient frequency to enable detection of every heartbeat and also refers to collection of heart rate data continuously throughout the day and night.

The term "pointing device," as used herein, refers to any suitable input interface, specifically, a human interface device, that allows a user to input spatial data to a computing system or device. In an exemplary embodiment, the pointing device may allow a user to provide input to the computer using physical gestures, for example, pointing, clicking, dragging, and dropping. Exemplary pointing devices may include, but are not limited to, a mouse, a touchpad, a touchscreen, and the like.

The term "multi-chip module," as used herein, refers to an electronic package in which multiple integrated circuits (IC) are packaged with a unifying substrate, facilitating their use as a single component, i.e., as a higher processing capacity IC packaged in a much smaller volume.

The term "computer-readable medium," as used herein, refers to a non-transitory storage hardware, non-transitory storage device or non-transitory computer system memory that may be accessed by a controller, a microcontroller, a computational system or a module of a computational system to encode thereon computer-executable instructions or software programs. The "computer-readable medium" may be accessed by a computational system or a module of a computational system to retrieve and/or execute the computer-executable instructions or software programs encoded on the medium. The non-transitory computer-readable media may include, but are not limited to, one or more types of hardware memory, non-transitory tangible media (for example, one or more magnetic storage disks, one or more optical disks, one or more USB flash drives), computer system memory or random access memory (such as, DRAM, SRAM, EDO RAM) and the like.

The term "distal," as used herein, refers to a portion, end or component of a physiological measurement system that is farthest from a user's body when worn by the user.

The term "proximal," as used herein, refers to a portion, end or component of a physiological measurement system that is closest to a user's body when worn by the user.

The term "equal," as used herein, refers, in a broad lay sense, to exact equality or approximate equality within some tolerance.

I. Exemplary Wearable Physiological Measurement Systems

Exemplary embodiments provide wearable physiological measurements systems that are configured to provide continuous measurement of heart rate. Exemplary systems are configured to be continuously wearable on an appendage, for example, wrist or ankle, and do not rely on electrocardiography or chest straps in detection of heart rate. The exemplary system includes one or more light emitters for emitting light at one or more desired frequencies toward the user's skin, and one or more light detectors for received light reflected from the user's skin. The light detectors may include a photo-resistor, a photo-transistor, a photo-diode, and the like. As light from the light emitters (for example, green light) pierces through the skin of the user, the blood's natural absorbance or transmittance for the light provides fluctuations in the photo-resistor readouts. These waves have the same frequency as the user's pulse since increased absorbance or transmittance occurs only when the blood flow has increased after a heartbeat. The system includes a processing module implemented in software, hardware or a combination thereof for processing the optical data received at the light detectors and continuously determining the heart rate based on the optical data. The optical data may be combined with data from one or more motion sensors, e.g., accelerometers and/or gyroscopes, to minimize or eliminate noise in the heart rate signal caused by motion or other artifacts (or with other optical data of another wavelength).

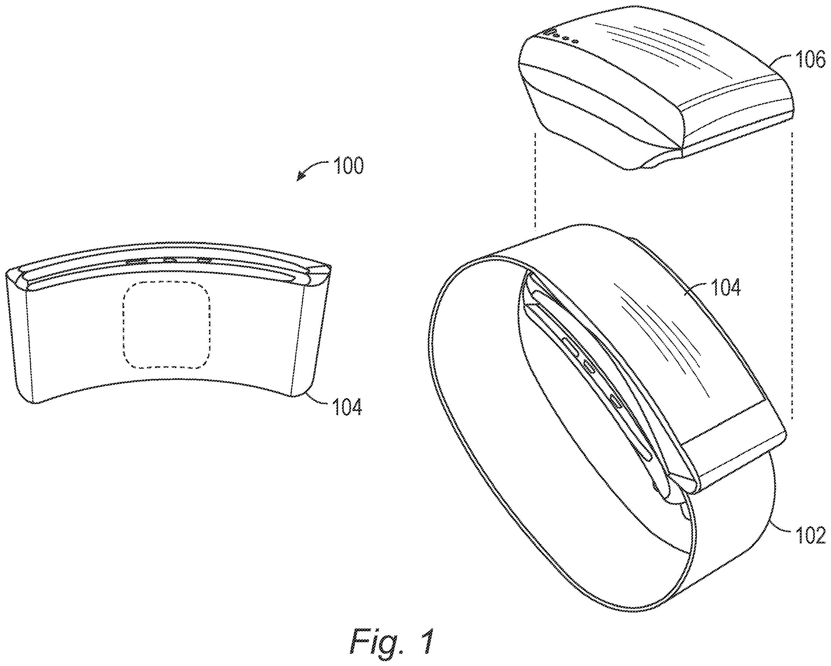

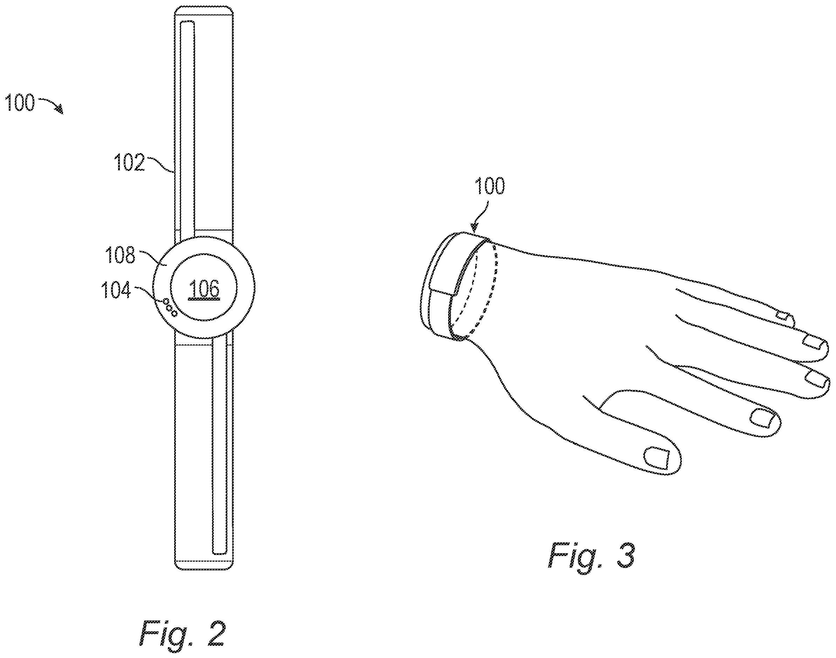

Fig. 1 illustrates front and back perspective views of one embodiment of a wearable system configured as a bracelet 100 including one or more straps 102. Fig. 2 shows another exemplary embodiment of a bracelet according to aspects disclosed herein with an exemplary user interface 106. The bracelet 100 may be sleek and lightweight, thereby making it appropriate for continuous wear. The bracelet 100 may or may not include a display screen, e.g., user interface 106 such as a light emitting diode (LED) display for displaying any desired data (e.g., instantaneous heart rate).

As shown in the non-limiting embodiment in Fig. 1, the strap 102 of the bracelet 100 may have a wider side and a narrower side. In one embodiment, a user may simply insert the narrower side into the thicker side and squeeze the two together until the strap 102 is tight around the wrist, as shown in Fig. 3. To remove the strap 102, a user may push the strap 102 further inwards, which unlocks the strap 102 and allows it to be released from the wrist. In other embodiments, various other fastening means may be provided. For example, the fastening mechanism may include, without limitation, a clasp, clamp, clip, dock, friction fit, hook and loop, latch, lock, pin, screw, slider, snap, button, spring, yoke, and so on.

In some embodiments, the strap 102 of the bracelet 100 may be a slim elastic band formed of any suitable elastic material, for example, rubber. Certain embodiments of the wearable system may be configured to have one size that fits all. Other embodiments may provide the ability to adjust for different wrist sizes. In one aspect, a combination of constant module strap material, a spring-loaded, floating optical system and a silicon-rubber finish may be used in order to achieve coupling while maintaining the strap's comfort for continuous use. Use of medical-grade materials to avoid skin irritations may be utilized.

As shown in Fig. 1, the wearable system (e.g., the bracelet 100) may include components configured to provide various functions such as data collection and streaming functions of the system. In some embodiments, the wearable system may include a button underneath the wearable system. In some embodiments, the button may be configured such that, when the wearable system is properly tightened to one's wrist, the button may press down and activate the system to begin storing information. In other embodiments, the button may be disposed and configured such that it may be pressed manually at the discretion of a user to begin storing information or otherwise to mark the start or end of an activity period. In some embodiments, the button may be held to initiate a time stamp and held again to end a time stamp, which may be transmitted, directly or through a mobile communication device application, to a website as a time stamp.

Time stamp information may be used, for example, as a privacy setting to indicate periods of activity during which physiological data may not be shared with other users. In one aspect, the button may be tapped, double-tapped (or triple-tapped or more), or held down in order to perform different functions or display different information (e.g., display battery information, generate time stamps, etc.). Other implementations may include more or less buttons or other forms of interfaces. More general, a privacy switch such as any of the user inputs or controls described herein may be operated to control restrictions on sharing, distribution, or use of heart rate or other continuously monitored physiological data. For example, the privacy switch may include a toggle switch to switch between a private setting where data is either not gathered at all or where data is stored locally for a user, and between a public, shared, or other non-private setting where data is communicated over a network and/or to a shared data repository. The privacy switch may also support numerous levels of privacy, e.g., using a hierarchical, role-based, and/or identity-based arrangement of permitted users and/or uses. As another example, various levels of privacy may be available for the type and amount of data that is shared versus private. In general, the privacy switch may be a physical switch on the wearable system, or a logical switch or the like maintained on a computer or other local or mobile computing device of the user, or on a website or other network-accessible resource where the user can select and otherwise control privacy settings for monitored physiological data.

In some embodiments, the wearable system may be waterproof so that users never need to remove it, thereby allowing for continuous wear.

The wearable system may include a heart rate monitor. In one example, the heart rate may be detected from the radial artery, in the exemplary positioning shown in Fig. 3. *See,* Certified Nursing Association, "Regular monitoring of your patient's radial pulse can help you detect changes in their condition and assist in providing potentially life-saving care." *See,* http://cnatraininghelp.com/cna-skills/counting-and-recording-a-radial-pulse. Thus, the wearable system may include a pulse sensor. In one embodiment, the wearable system may be configured such that, when a user wears it around their wrist and tightens it, the sensor portion of the wearable system is secured over the user's radial artery or other blood vessel. Secure connection and placement of the pulse sensor over the radial artery or other blood vessel may allow measurement of heart rate and pulse. It will be understood that this configuration is provided by way of example only, and that other sensors, sensor positions, and monitoring techniques may also or instead be employed without departing from the scope of this disclosure.

In some embodiments, the pulse or heart rate may be taken using an optical sensor coupled with one or more light emitting diodes (LEDs), all directly in contact with the user's wrist. The LEDs are provided in a suitable position from which light can be emitted into the user's skin. In one example, the LEDs mounted on a side or top surface of a circuit board in the system to prevent heat buildup on the LEDs and to prevent burns on the skin. The circuit board may be designed with the intent of dissipating heat, e.g., by including thick conductive layers, exposed copper, heatsink, or similar. In one aspect, the pulse repetition frequency is such that the amount of power thermally dissipated by the LED is negligible. Cleverly designed elastic wrist straps can ensure that the sensors are always in contact with the skin and that there is a minimal amount of outside light seeping into the sensors. In addition to the elastic wrist strap, the design of the strap may allow for continuous micro adjustments (no preset sizes) in order to achieve an optimal fit, and a floating sensor module. The sensor module may be free to move with the natural movements caused by flexion and extension of the wrist.

In some embodiments, the wearable system may be configured to record other physiological parameters including, but not limited to, skin temperature (using a thermometer), galvanic skin response (using a galvanic skin response sensor), motion (using one or more multi-axes accelerometers and/or gyroscope), and the like, and environmental or contextual parameters, e.g., ambient temperature, humidity, time of day, and the like. In an implementation, sensors are used to provide at least one of continuous motion detection, environmental temperature sensing, electrodermal activity (EDA) sensing, galvanic skin response (GSR) sensing, and the like. In this manner, an implementation can identify the cause of a detected physiological event. Reflectance PhotoPlethysmoGraphy (RPPG) may be used for the detection of cardiac activity, which may provide for non-intrusive data collection, usability in wet, dusty and otherwise harsh environments, and low power requirements. For example, as explained herein, using the physiological readouts of the device and the analytics described herein, an "Intensity Score" (e.g., 0-21) (e.g., that measures a user's recent exertion), a "Recovery Score" (e.g., 0-100%), and "Sleep Score" (e.g., 0-100) may together measure readiness for physical and psychological exertion.

In some embodiments, the wearable system may further be configured such that a button underneath the system may be pressed against the user's wrist, thus triggering the system to begin one or more of collecting data, calculating metrics and communicating the information to a network. In some embodiments, the sensor used for, e.g., measuring heart rate or GSR or any combination of these, may be used to indicate whether the user is wearing the wearable system or not. In some embodiments, power to the one or more LEDs may be cut off as soon as this situation is detected and reset once the user has put the wearable system back on their wrist.

The wearable system may include one, two, or more sources of battery life, e.g., two or more batteries. In some embodiments, it may have a battery that can slip in and out of the head of the wearable system and can be recharged using an included accessory. Additionally, the wearable system may have a built-in battery that is less powerful. When the more powerful battery is being charged, the user does not need to remove the wearable system and can still record data (during sleep, for example).

In some embodiments, an application associated with data from an exemplary wearable system (e.g., a mobile communication device application) may include a user input component for enabling additional contextual data, e.g., emotional (e.g., the user's feelings), perceived intensity, and the like. When the data is uploaded from the wearable system directly or indirectly to a website, the website may record a user's "Vibes" alongside their duration of exercise and sleep.

In exemplary embodiments, the wearable system is enabled to automatically detect when the user is asleep, awake but at rest and exercising based on physiological data collected by the system.

As shown in Fig. 2, a rotatable wheel 108 may be provided at the substantial center (or elsewhere) of the wearable system to control whether the system is displaying the heart rate. For example, when the wheel is turned to the right however, the system continuously shows heart rate, and turns off the heart rate display when the wheel is turned to the right again. In one example, turning the wheel to the right and holding it there creates a time stamp to indicate the duration of exercise. Turning the wheel to the left and holding it there forces data transmission to a cell phone, external computer or the Internet. In other embodiments, the wheel 108 may be absent in the wearable system. In some embodiments, the functionality of a rotatable wheel 108 described herein may be provided in an application of a mobile communication device that is associated with physiological data collected from a wearable system.

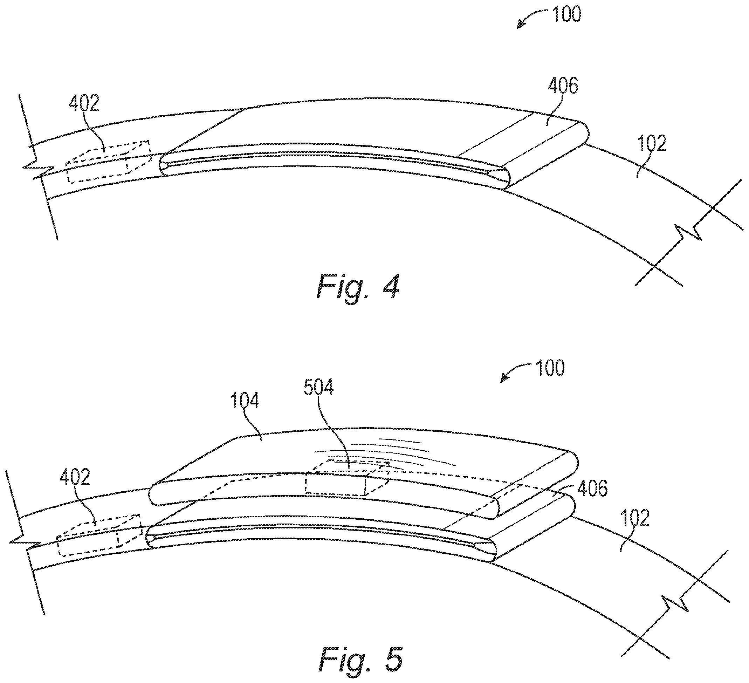

In some embodiments, a physiological measurement system may be configured in a modular design to enable continuous operation of the system in monitoring physiological information of a user wearing the system. The module design may include a strap and a separate modular head portion or housing that is removably couplable to the strap. Fig. 4 illustrates a side view of an exemplary physiological measurement system 100 including a strap 102 that is not coupled to a modular head portion or housing 104. Fig. 5 illustrates a side view of the system 100 in which the modular head portion 104 is removably coupled to the strap 102.

In the non-limiting illustrative module design, the strap 102 of a physiological measurement system may be provided with a set of components that enables continuous monitoring of at least a heart rate of the user so that it is independent and fully self-sufficient in continuously monitoring the heart rate without requiring the modular head portion 104. In one embodiment, the strap includes a plurality of light emitters for emitting light toward the user's skin, a plurality of light detectors for receiving light reflected from the user's skin, an electronic circuit board comprising a plurality of electronic components configured for analyzing data corresponding to the reflected light to automatically and continually determine a heart rate of the user, and a first set of one or more batteries for supplying electrical power to the light emitters, the light detectors and the electronic circuit board. In some embodiments, the strap may also detect one or more other physiological characteristics of the user including, but not limited to, temperature, galvanic skin response, and the like. The strap may include one or more slots for permanently or removably coupling batteries 402 to the strap 102.

The strap 102 may include an attachment mechanism 406, e.g., a press-fit mechanism, for coupling the modular head portion 104 to the strap 102. The modular head portion 104 may be coupled to the strap 102 at any desired time by the user to impart additional functionality to the system 100. In one embodiment, the modular head portion 104 includes a second set of one or more batteries 504 chargeable by an external power source so that the second set of batteries can be used to charge or recharge the first set of batteries 402 in the strap 102. The combination of the first and second sets of batteries enables the user to continuously monitor his/her physiological information without having to remove the strap for recharging. In some embodiments, the module head portion may include one or more additional components including, but not limited to, an interface 616 including visual display device configured to render a user interface for displaying physiological information of the user, a GPS sensor, an electronic circuit board (e.g., to process GPS signals), and the like.

Certain exemplary systems may be configured to be coupled to any desired part of a user's body so that the system may be moved from one portion of the body (e.g., wrist) to another portion of the body (e.g., ankle) without affecting its function and operation. An exemplary system may include an electronic circuit board comprising a plurality of electronic components configured for analyzing data corresponding to the reflected light to automatically and continually determine a heart rate of the user. The electronic circuit board implements a processing module configured to detect an identity of a portion of the user's body, for example, an appendage like wrist, ankle, to which the strap is coupled based on one or more signals associated with the heart rate of the user, and, based on the identity of the appendage, adjust data analysis of the reflected light to determine the heart rate of the user.

In one embodiment, the identity of the portion of the user's body to which the wearable system is attached may be determined based on one or more parameters including, but not limited to, absorbance level of light as returned from the user's skin, reflectance level of light as returned from the user's skin, motion sensor data (e.g., accelerometer and/or gyroscope), altitude of the wearable system, and the like.

In some embodiments, the processing module is configured to determine that the wearable system is taken off from the user's body. In one example, the processing module may determine that the wearable system has been taken off if data from the galvanic skin response sensor indicates data atypical of a user's skin. If the wearable system is determined to be taken off from the user's body, the processing module is configured to deactivate the light emitters and the light detectors and cease monitoring of the heart rate of the user to conserve power.

In some exemplary embodiments, the electronic components of the physiological measurement system may be provided in the form of a multi-chip module in which a plurality of electrically-coupled electronic circuit boards are provided separately within the system. In one non-limiting example, the processor and random-access memory (RAM) may be provided on a first circuit board, wireless communication components may be provided on a second circuit board, and sensors may be provided on a third circuit board. The separate electronic circuit boards may be provided in a modular head of the system and/or along a strap of the system. The term "multi-chip module," as used herein, refers to an electronic package in which multiple integrated circuits (IC) are packaged with a unifying substrate, facilitating their use as a single component, i.e., as a higher processing capacity IC packaged in a much smaller volume. Each IC can comprise a circuit fabricated in a thinned semiconductor wafer. Any suitable set of one or more electronic components may be provided in the circuit boards of a multi-chip module. Exemplary embodiments also provide methods for fabricating and assembling multi-chip modules as taught herein.

Exemplary numbers of chips integrated in a multi-chip module may include, but are not limited to, two, three, four, five, six, seven, eight, and the like. In one embodiment of a physiological measurement system, a single multi-chip module is provided on a circuit board that performs operations to generate physiological information associated with a user of the system. In other embodiments, a plurality of multi-chip modules are provided on a circuit board of the physiological measurement system. The plurality of multi-chip modules may be stacked vertically on top of one another on the circuit board to further minimize the packaging size and the footprint of the circuit board.

In one multi-chip embodiment, two or more electrically-coupled circuit boards of a multi-chip module may be provided in a physiological measurement system in a vertically stacked manner to minimize the packaging size and the footprint of the circuit board. Vertically stacking the components on a circuit board minimizes the packaging size (e.g., the length and width) and the footprint occupied by the chips on the circuit board. In certain non-limiting embodiments, a circuit board including one or more physiological sensors may be placed closest to, proximal to or in contact with the user's skin, while one or more circuit boards including one or more processors, storage devices, communication components and non-physiological sensors may be provided in vertical layers that are distal to the user's skin.

Exemplary systems include a processing module configured to filter the raw photoplethysmography data received from the light detectors to minimize contributions due to motion, and subsequently process the filtered data to detect peaks in the data that correspond with heart beats of a user. The overall algorithm for detecting heart beats takes as input the analog signals from optical sensors (mV) and accelerometer, and outputs an implied beats per minute (heart rate) of the signal accurate within a few beats per minute as that determined by an electrocardiography machine even during motion.

In one aspect, using multiple LEDs with different wavelengths reacting to movement in different ways can allow for signal recovery with standard signal processing techniques. The availability of accelerometer information can also be used to compensate for coarse movement signal corruption. In order to increase the range of movements that the algorithm can successfully filter out, an aspect utilizes techniques that augment the algorithm already in place. For example, filtering violent movements of the arm during very short periods of time, such as boxing as exercising, may be utilized by the system. By selective sampling and interpolating over these impulses, an aspect can account for more extreme cases of motion. Additionally, an investigation into different LED wavelengths, intensities, and configurations can allow the systems described herein to extract a signal across a wide spectrum of skin types and wrist sizes. In other words, motion filtering algorithms and signal processing techniques may assist in mitigating the risk caused by movement.

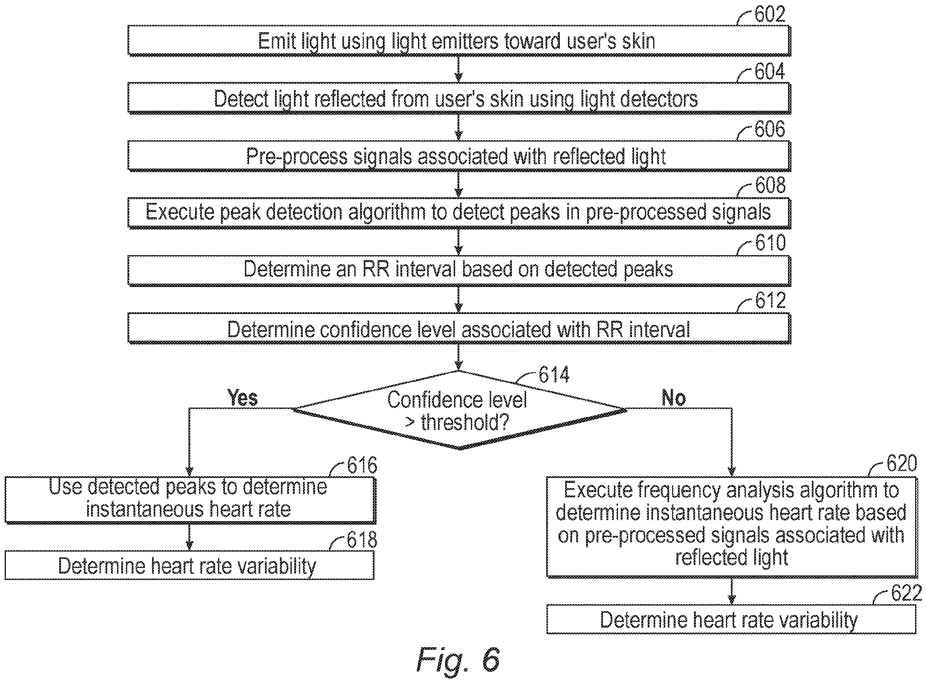

Fig. 6 is a flow chart illustrating an exemplary signal processing algorithm for generating a sequence of heart rates for every detected heartbeat that is embodied in computer-executable instructions stored on one or more non-transitory computer-readable media. In step 602, light emitters of a wearable physiological measurement system emit light toward a user's skin. In step 604, light reflected from the user's skin is detected at the light detectors in the system. In step 606, signals or data associated with the reflected light are pre-processed using any suitable technique to facilitate detection of heart beats. In step 608, a processing module of the system executes one or more computer-executable instructions associated with a peak detection algorithm to process data corresponding to the reflected light to detect a plurality of peaks associated with a plurality of beats of the user's heart. In step 610, the processing module determines an RR interval based on the plurality of peaks detected by the peak detection algorithm. In step 612, the processing module determines a confidence level associated with the RR interval.

Based on the confidence level associated with the RR interval estimate, the processing module selects either the peak detection algorithm or a frequency analysis algorithm to process data corresponding to the reflected light to determine the sequence of instantaneous heart rates of the user. The frequency analysis algorithm may process the data corresponding to the reflected light based on the motion of the user detected using, for example, an accelerometer. The processing module may select the peak detection algorithm or the frequency analysis algorithm regardless of a motion status of the user. It is advantageous to use the confidence in the estimate in deciding whether to switch to frequency-based methods as certain frequency-based approaches are unable to obtain accurate RR intervals for heart rate variability analysis. Therefore, an implementation maintains the ability to obtain the RR intervals for as long as possible, even in the case of motion, thereby maximizing the information that can be extracted.

For example, in step 614, it is determined whether the confidence level associated with the RR interval is above (or equal to or above) a threshold. In certain embodiments, the threshold may be predefined, for example, about 50%-90% in some embodiments and about 80% in one non-limiting embodiment. In other embodiments, the threshold may be adaptive, i.e., the threshold may be dynamically and automatically determined based on previous confidence levels. For example, if one or more previous confidence levels were high (i.e., above a certain level), the system may determine that a present confidence level that is relatively low compared to the previous levels is indicative of a less reliable signal. In this case, the threshold may be dynamically adjusted to be higher so that a frequency-based analysis method may be selected to process the less reliable signal.

If the confidence level is above (or equal to or above) the threshold, in step 616, the processing module may use the plurality of peaks to determine an instantaneous heart rate of the user. On the other hand, in step 620, based on a determination that the confidence level associated with the RR interval is equal to or below the predetermined threshold, the processing module may execute one or more computer-executable instructions associated with the frequency analysis algorithm to determine an instantaneous heart rate of the user. The confidence threshold may be dynamically set based on previous confidence levels.

In some embodiments, in steps 618 or 622, the processing module determines a heart rate variability of the user based on the sequence of the instantaneous heart rates/beats.

The system may include a display device configured to render a user interface for displaying the sequence of the instantaneous heart rates of the user, the RR intervals and/or the heart rate variability determined by the processing module. The system may include a storage device configured to store the sequence of the instantaneous heart rates, the RR intervals and/or the heart rate variability determined by the processing module.

In one aspect, the system may switch between different analytical techniques for determining a heart rate such as a statistical technique for detecting a heart rate and a frequency domain technique for detecting a heart rate. These two different modes have different advantages in terms of accuracy, processing efficiency, and information content, and as such may be useful at different times and under different conditions. Rather than selecting one such mode or technique as an attempted optimization, the system may usefully switch back and forth between these differing techniques, or other analytical techniques, using a predetermined criterion. For example, where statistical techniques are used, a confidence level may be determined and used as a threshold for switching to an alternative technique such as a frequency domain technique. The threshold may also or instead depend on historical, subjective, and/or adapted data for a particular user. For example, selection of a threshold may depend on data for a particular user including without limitation subjective information about how a heart rate for a particular user responds to stress, exercise, and so forth. Similarly, the threshold may adapt to changes in fitness of a user, context provided from other sensors of the wearable system, signal noise, and so forth.

An exemplary statistical technique employs probabilistic peak detection. In this technique, a discrete probabilistic step may be set, and a likelihood function may be established as a mixture of a Gaussian random variable and a uniform. The heart of the likelihood function encodes the assumption that with a first probability (*p*) the peak detection algorithm has produced a reasonable initial estimate, but with a second probability (1-*p*) it has not. In a subsequent step, Bayes' rule is applied to determine the posterior density on the parameter space, of which the maximum is taken (that is, the argument (parameter) that maximizes the posterior distribution). This value is the estimate for the heart rate. In a subsequent step, the previous two steps are reapplied for the rest of the sample. There is some variance in the signal due to process noise, which is dependent on the length of the interval. This process noise becomes the variance in the Gaussians used for the likelihood function. Then, the estimate is obtained as the maximum a posteriori on the new posterior distribution. A confidence value is recorded for the estimate which, for some precision measurement, the posterior value is summed at points in the parameter space centered at our estimate +/- the precision.

The beats per minute (BPM) parameter space, $\theta$, may range between about 20 and about 240, corresponding to the empirical bounds on human heart rates. In an exemplary method, a probability distribution is calculated over this parameter space, at each step declaring the mode of the distribution to be the heart rate estimate. A discrete uniform prior may be set:

$$\pi_1 \sim \mathrm{DiscUnif}(\theta)$$

The un-normalized, univariate likelihood is defined by a mixture of a Gaussian function and a uniform:

$$l_1 \sim IG + (1 - I)U, G \sim N(\gamma_1\sigma^2), I \sim \mathrm{Ber(p)}$$

where

$U \sim \mathrm{DiscUnif}(\theta)$
and where $\sigma$ and *p* are predetermined constants.

Bayes' rule is applied to determine the posterior density on θ, for example, by component-wise multiplying the prior density vector $(\pi_1(\theta))_{\theta \in \theta}$ with the likelihood vector $(l_1(\theta))_{\theta \in \theta}$ to obtain the posterior distribution $\eta_1$. Then, the following is set:

$$\beta_1 = \mathrm{argmax}_{\theta \epsilon \theta}\, \eta_1(\theta)$$

For k≥2, the variance in signal S(t) due to process noise is determined. Then, the following variable is set to imbue temporally long RR intervals with more process/interpeak noise and set the post-normalization convolution:

$$\pi_k = \eta_{k-1} * f_{N\left(o,\lambda_k^2\right)|\theta}$$

where *f* is a density function of the following:

$$Z \sim N\left(o, \lambda_k^2\right)$$

Then, the following expressions are calculated:

$$l_k \sim pG_k + (1-p)U, \;\; G_k \sim N(\lambda k, \sigma^2)$$

The expression is then normalized and recorded:

$$\beta_k = \mathrm{argmax}_{\theta \epsilon \theta}\, \eta_k(\theta)$$

Finally, the confidence level of the above expression for a particular precision threshold is determined:

$$C_k = \textstyle\sum_{\theta \epsilon [\beta_k - e_1, \beta_k + e] \cap \theta}\, \eta_k.$$

An exemplary frequency analysis algorithm used in an implementation isolates the highest frequency components of the optical data, checks for harmonics common in both the accelerometer data and the optical data, and performs filtering of the optical data. The algorithm takes as input raw analog signals from the accelerometer (3-axis) and pulse sensors, and outputs heart rate values or beats per minute (BPM) for a given period of time related to the window of the spectrogram.

The isolation of the highest frequency components is performed in a plurality of stages, gradually winnowing the window-sizes of consideration, thereby narrowing the range of errors. In one implementation, a spectrogram of 2^15 samples with overlap 2^13 samples of the optical data is generated. The spectrogram is restricted to frequencies in which heart rate can lie. These restriction boundaries may be updated when smaller window sizes are considered. The frequency estimate is extracted from the spectrogram by identifying the most prominent frequency component of the spectrogram for the optical data. The frequency may be extracted using the following exemplary steps. The most prominent frequency of the spectrogram is identified in the signal. It is determined if the frequency estimate is a harmonic of the true frequency. The frequency estimate is replaced with the true frequency if the estimate is a harmonic of the true frequency. It is determined if the current frequency estimate is a harmonic of the motion sensor data. The frequency estimate is replaced with a previous temporal estimate if it is a harmonic of the motion sensor data. The upper and lower bounds on the frequency obtained are saved. A constant value may be added or subtracted in some cases. In subsequent steps, the constant added or subtracted may be reduced to provide narrower searches. A number of the previous steps are repeated one or more times, e.g., three times, except taking $2^{15-i}$ samples for the window size and $2^{13-i}$ for the overlap in the spectrogram where i is the current number of iteration. The final output is the average of the final symmetric endpoints of the frequency estimation.

The table below demonstrates the performance of the algorithm disclosed herein. To arrive at the results below, experiments were conducted in which a subject wore an exemplary wearable physiological measurement system and a 3-lead ECG which were both wired to the same microcontroller (e.g., Arduino) in order to provide time-synced data. Approximately 50 data sets were analyzed which included the subject standing still, walking, and running on a treadmill.

Table 1: Performance of signal processing algorithm disclosed herein

|  | Clean data error (mean, std) in BPM | Noisy data error (mean, std) in BPM |
|---|---|---|
| 4-level spectrogram (80 second blocks) | 0.2, 2.3 | 0.8, 5.1 |

The algorithm's performance comes from a combination of a probabilistic and frequency based approach. The three difficulties in creating algorithms for heart rate calculations from the PPG data are 1) false detections of beats, 2) missed detections of real beats, and 3) errors in the precise timing of the beat detection. The algorithms disclosed herein provide improvements in these three sources of error and, in some cases, the error is bound to within 2 BPM of ECG values at all times even during the most motion intense activities.

The exemplary wearable system computes heart rate variability (HRV) to obtain an understanding of the recovery status of the body. These values are captured right before a user awakes or when the user is not moving, in both cases photoplethysmography (PPG) variability yielding equivalence to the ECG HRV. HRV is traditionally measured using an ECG machine and obtaining a time series of R-R intervals. Because an exemplary wearable system utilizes photoplethysmography (PPG), it does not obtain the electric signature from the heart beats; instead, the peaks in the obtained signal correspond to arterial blood volume. At rest, these peaks are directly correlated with cardiac cycles, which enables the calculation of HRV via analyzing peak-to-peak intervals (the PPG analog of RR intervals). It has been demonstrated in the medical literature that these peak-to-peak intervals, the "PPG variability," is identical to ECG HRV while at rest. *See,* Chariot K, et al. "Interchangeability between heart rate and photoplethysmography variabilities during sympathetic stimulations." Physiological Measurement. 2009 Dec; 30(12): 1357-69. doi: 10.1088/0967-3334/30/12/005. URL: http://www.ncbi.nlm.nih.gov/pubmed/19864707; and Lu, S, et. al. "Can photoplethysmography variability serve as an alternative approach to obtain heart rate variability information?" Journal of Clinical Monitoring and Computing. 2008 Feb; 22(1):23-9. URL: http://www.ncbi.nlm.nih.gov/pubmed/17987395.

Exemplary physiological measurement systems are configured to minimize power consumption so that the systems may be worn continuously without requiring power recharging at frequent intervals. The majority of current draw in an exemplary system is allocated to power the light emitters, e.g., LEDs, the wireless transceiver, the microcontroller and peripherals. In one embodiment, the circuit board of the system may include a boost converter that runs a current of about 10 mA through each of the light emitters with an efficiency of about 80% and may draw power directly from the batteries at substantially constant power. With exemplary batteries at about 3.7 V, the current draw from the battery may be about 40 mW. In some embodiments, the wireless transceiver may draw about 10-20 mA of current when it is actively transferring data. In some embodiments, the microcontroller and peripherals may draw about 5 mA of current.

An exemplary system may include a processing module that is configured to automatically adjust one or more operational characteristics of the light emitters and/or the light detectors to minimize power consumption while ensuring that all heart beats of the user are reliably and continuously detected. The operational characteristics may include, but are not limited to, a frequency of light emitted by the light emitters, the number of light emitters activated, a duty cycle of the light emitters, a brightness of the light emitters, a sampling rate of the light detectors, and the like.

The processing module may adjust the operational characteristics based on one or more signals or indicators obtained or derived from one or more sensors in the system including, but not limited to, a motion status of the user, a sleep status of the user, historical information on the user's physiological and/or habits, an environmental or contextual condition (e.g., ambient light conditions), a physical characteristic of the user (e.g., the optical characteristics of the user's skin), and the like.

In one embodiment, the processing module may receive data on the motion of the user using, for example, an accelerometer. The processing module may process the motion data to determine a motion status of the user which indicates the level of motion of the user, for example, exercise, light motion (e.g., walking), no motion or rest, sleep, and the like. The processing module may adjust the duty cycle of one or more light emitters and the corresponding sampling rate of the one or more light detectors based on the motion status. For example, upon determining that the motion status indicates that the user is at a first higher level of motion, the processing module may activate the light emitters at a first higher duty cycle and sample the reflected light using light detectors sampling at a first higher sampling rate. Upon determining that the motion status indicates that the user is at a second lower level of motion, the processing module may activate the light emitters at a second lower duty cycle and sample the reflected light using light detectors sampling at a second lower sampling rate. That is, the duty cycle of the light emitters and the corresponding sampling rate of the light detectors may be adjusted in a graduated or continuous manner based on the motion status or level of motion of the user. This adjustment ensures that heart rate data is detected at a sufficiently high frequency during motion to reliably detect all of the heart beats of the user.

In non-limiting examples, the light emitters may be activated at a duty cycle ranging from about 1% to about 100%. In another example, the light emitters may be activated at a duty cycle ranging from about 20% to about 50% to minimize power consumption. Certain exemplary sampling rates of the light detectors may range from about 50 Hz to about 1000 Hz, but are not limited to these exemplary rates. Certain non-limiting sampling rates are, for example, about 100 Hz, 200 Hz, 500 Hz, and the like.

In one non-limiting example, the light detectors may sample continuously when the user is performing an exercise routine so that the error standard deviation is kept within 5 beats per minute (BPM). When the user is at rest, the light detectors may be activated for about a 1% duty cycle-10 milliseconds each second (i.e., 1% of the time) so that the error standard deviation is kept within 5 BPM (including an error standard deviation in the heart rate measurement of 2 BPM and an error standard deviation in the heart rate changes between measurement of 3 BPM). When the user is in light motion (e.g., walking), the light detectors may be activated for about a 10% duty cycle-100 milliseconds each second (i.e., 10% of the time) so that the error standard deviation is kept within 6 BPM (including an error standard deviation in the heart rate measurement of 2 BPM and an error standard deviation in the heart rate changes between measurement of 4 BPM).

The processing module may adjust the brightness of one or more light emitters by adjusting the current supplied to the light emitters. For example, a first level of brightness may be set by current ranging between about 1 mA to about 10 mA, but is not limited to this exemplary range. A second higher level of brightness may be set by current ranging from about 11 mA to about 30 mA, but is not limited to this exemplary range. A third higher level of brightness may be set by current ranging from about 80 mA to about 120 mA, but is not limited to this exemplary range. In one non-limiting example, first, second and third levels of brightness may be set by current of about 5 mA, about 20 mA and about 100 mA, respectively.

In some embodiments, the processing module may detect an environmental or contextual condition (e.g., level of ambient light) and adjust the brightness of the light emitters accordingly to ensure that the light detectors reliably detect light reflected from the user's skin while minimizing power consumption. For example, if it is determined that the ambient light is at a first higher level, the brightness of the light emitters may be set at a first higher level. If it is determined that the ambient light is at a second lower level, the brightness of the light emitters may be set at a second lower level. In some cases, the brightness may be adjusted in a continuous manner based on the detected environment condition.

In some embodiments, the processing module may detect a physiological condition of the user (e.g., an optical characteristic of the user's skin) and adjust the brightness of the light emitters accordingly to ensure that the light detectors reliably detect light reflected from the user's skin while minimizing power consumption. For example, if it is determined that the user's skin is highly reflective, the brightness of the light emitters may be set at a first lower level. If it is determined that the user's skin is not very reflective, the brightness of the light emitters may be set at a second higher level.

Shorter-wavelength LEDs may require more power than is required by longer-wavelength LEDs. Therefore, an exemplary wearable system may provide and use light emitted at two or more different frequencies based on the level of motion detected in order to save battery life. For example, upon determining that the motion status indicates that the user is at a first higher level of motion (e.g., exercising), one or more light emitters may be activated to emit light at a first wavelength. Upon determining that the motion status indicates that the user is at a second lower level of motion (e.g., at rest), one or more light emitters may be activated to emit light at a second wavelength that is longer than the first wavelength. Upon determining that the motion status indicates that the user is at a third lower level of motion (e.g., sleeping), one or more light emitters may be activated to emit light at a third wavelength that is longer than the first and second wavelengths. Other levels of motion may be predetermined and corresponding wavelengths of emitted light may be selected. The threshold levels of motion that trigger adjustment of the light wavelength may be based on one or more factors including, but are not limited to, skin properties, ambient light conditions, and the like. Any suitable combination of light wavelengths may be selected, for example, green (for a higher level of motion)/red (for a lower level of motion); red (for a higher level of motion)/infrared (for a lower level of motion); blue (for a higher level of motion)/green (for a lower level of motion); and the like.

Shorter-wavelength LEDs may require more power than is required by other types of heart rate sensors, such as, a piezo-sensor or an infrared sensor. Therefore, an exemplary wearable system may provide and use a unique combination of sensors-one or more light detectors for periods where motion is expected and one or more piezo and/or infrared sensors for low motion periods (e.g., sleep)-to save battery life. Certain other embodiments of a wearable system may exclude piezo-sensors and/or infrared sensors.

For example, upon determining that the motion status indicates that the user is at a first higher level of motion (e.g., exercising), one or more light emitters may be activated to emit light at a first wavelength. Upon determining that the motion status indicates that the user is at a second lower level of motion (e.g., at rest), non-light based sensors may be activated. The threshold levels of motion that trigger adjustment of the type of sensor may be based on one or more factors including, but are not limited to, skin properties, ambient light conditions, and the like.

The system may determine the type of sensor to use at a given time based on the level of motion (e.g., via an accelerometer) and whether the user is asleep (e.g., based on movement input, skin temperature and heart rate). Based on a combination of these factors the system selectively chooses which type of sensor to use in monitoring the heart rate of the user. Common symptoms of being asleep are periods of no movement or small bursts of movement (such as shifting in bed), lower skin temperature (although it is not a dramatic drop from normal), drastic GSR changes, and heart rate that is below the typical resting heart rate when the user is awake. These variables depend on the physiology of a person and thus a machine learning algorithm is trained with user-specific input to determine when he/she is awake/asleep and determine from that the exact parameters that cause the algorithm to deem someone asleep.

In an exemplary configuration, the light detectors may be positioned on the underside of the wearable system and all of the heart rate sensors may be positioned adjacent to each other. For example, the low power sensor(s) may be adjacent to the high power sensor(s) as the sensors may be chosen and placed where the strongest signal occurs. In one example configuration, a 3-axis accelerometer may be used that is located on the top part of the wearable system.

In some embodiments, the processing module may be configured to automatically adjust a rate at which data is transmitted by the wireless transmitter to minimize power consumption while ensuring that raw and processed data generated by the system is reliably transmitted to external computing devices. In one embodiment, the processing module determines an amount of data to be transmitted (e.g., based on the amount of data generated since the time of the last data transmission), and may select the next data transmission time based on the amount of data to be transmitted. For example, if it is determined that the amount of data exceeds (or is equal to or greater than) a threshold level, the processing module may transmit the data or may schedule a time for transmitting the data. On the other hand, if it is determined that the amount of data does not exceed (or is equal to or lower than) the threshold level, the processing module may postpone data transmission to minimize power consumption by the transmitter. In one non-limiting example, the threshold may be set to the amount of data that may be sent in two seconds under current conditions. Exemplary data transmission rates may range from about 50kbytes per second to about 1 MByte per second, but are not limiting to this exemplary range.

In some embodiments, an operational characteristic of the microprocessor may be automatically adjusted to minimize power consumption. This adjustment may be based on a level of motion of the user's body.

More generally, the above description contemplates a variety of techniques for sensing conditions relating to heart rate monitoring or related physiological activity either directly (e.g., confidence levels or accuracy of calculated heart rate) or indirectly (e.g., motion detection, temperature). However measured, these sensed conditions can be used to intelligently select from among a number of different modes, including hardware modes, software modes, and combinations of the foregoing, for monitoring heart rate based on, e.g., accuracy, power usage, detected activity states, and so forth. Thus there is disclosed herein techniques for selecting from among two or more different heart rate monitoring modes according to a sensed condition.

II. Exemplary Physiological Analytics System

Exemplary embodiments provide an analytics system for providing qualitative and quantitative monitoring of a user's body, health and physical training. The analytics system is implemented in computer-executable instructions encoded on one or more non-transitory computer-readable media. The analytics system relies on and uses continuous data on one or more physiological parameters including, but not limited to, heart rate. The continuous data used by the analytics system may be obtained or derived from an exemplary physiological measurement system disclosed herein, or may be obtained or derived from a derived source or system, for example, a database of physiological data. In some embodiments, the analytics system computes, stores and displays one or more indicators or scores relating to the user's body, health and physical training including, but not limited to, an intensity score and a recovery score. The scores may be updated in real-time and continuously or at specific time periods, for example, the recovery score may be determined every morning upon waking up, the intensity score may be determined in real-time or after a workout routine or for an entire day.

In certain exemplary embodiments, a fitness score may be automatically determined based on the physiological data of two or more users of exemplary wearable systems.

An intensity score or indicator provides an accurate indication of the cardiovascular intensities experienced by the user during a portion of a day, during the entire day or during any desired period of time (e.g., during a week or month). The intensity score is customized and adapted for the unique physiological properties of the user and takes into account, for example, the user's age, gender, anaerobic threshold, resting heart rate, maximum heart rate, and the like.

If determined for an exercise routine, the intensity score provides an indication of the cardiovascular intensities experienced by the user continuously throughout the routine. If determined for a period of including and beyond an exercise routine, the intensity score provides an indication of the cardiovascular intensities experienced by the user during the routine and also the activities the user performed after the routine (e.g., resting on the couch, active day of shopping) that may affect their recovery or exercise readiness.

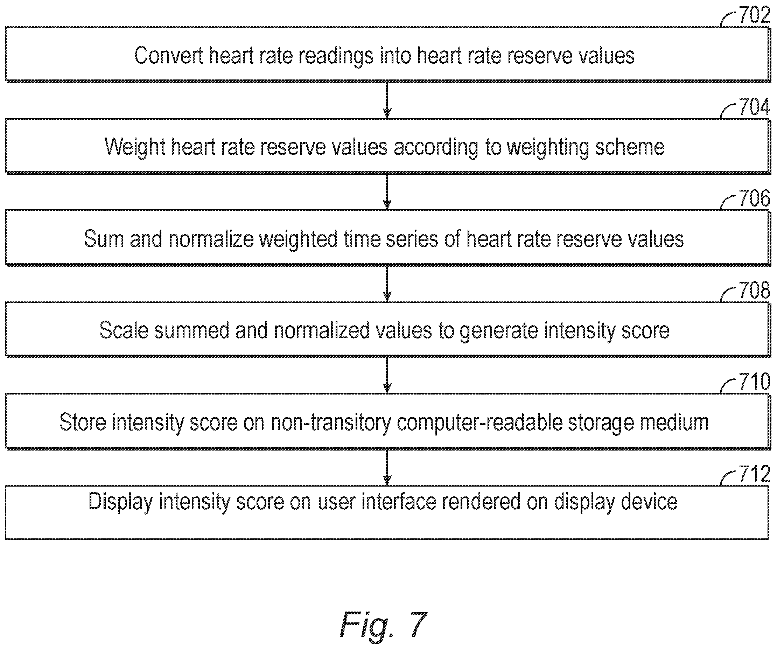

In exemplary embodiments, the intensity score is calculated based on the user's heart rate reserve (HRR) as detected continuously throughout the desired time period, for example, throughout the entire day. In one embodiment, the intensity score is an integral sum of the weighted HRR detected continuously throughout the desired time period. Fig. 7 is a flow chart illustrating an exemplary method of determining an intensity score.

In step 702, continuous heart rate readings are converted to HRR values. A time series of heart rate data used in step 702 may be denoted as:

$$H \in T$$

A time series of HRR measurements, v(t), may be defined in the following expression in which MHR is the maximum heart rate and RHR is the resting heart rate of the user:

$$v(t) = \frac{H(t) - RHR}{MHR - RHR}$$

In step 704, the HRR values are weighted according to a suitable weighting scheme. Cardiovascular intensity, indicated by an intensity score, is defined in the following expression in which w is a weighting function of the HRR measurements:

$$I(t_o, t_1) = \int_{t_0}^{t_1} w\big(v(t)\big) dt$$

In step 706, the weighted time series of HRR values is summed and normalized.

$$I_t = \int_T w\big(v(t)\big) dt \leq w(1)|T|$$

Thus, the weighted sum is normalized to the unit interval, i.e., [0, 1]:

$$N_T = \frac{I_T}{w(1) \cdot 24\text{hr}}$$

In step 708, the summed and normalized values are scaled to generate user-friendly intensity score values. That is, the unit interval is transformed to have any desired distribution in a scale (e.g., a scale including 21 points from 0 to 21), for example, arctangent, sigmoid, sinusoidal, and the like. In certain distributions, the intensity values increase at a linear rate along the scale, and in others, at the highest ranges the intensity values increase at more than a linear rate to indicate that it is more difficult to climb in the scale toward the extreme end of the scale. In some embodiments, the raw intensity scores are scaled by fitting a curve to a selected group of "canonical" exercise routines that are predefined to have particular intensity scores.

In one embodiment, monotonic transformations of the unit interval are achieved to transform the raw HRR values to user-friendly intensity scores. An exemplary scaling scheme, expressed as f: [0, 1] → [0, 1], is performed using the following function:

$$(x, N, p) = 0.5 \left( \frac{\arctan(N(x-p))}{\pi/2} + 1 \right)$$

To generate an intensity score, the resulting value may be multiplied by a number based on the desired scale of the intensity score. For example, if the intensity score is graduated from zero to 21, then the value may be multiplied by 21.

In step 710, the intensity score values are stored on a non-transitory storage medium for retrieval, display and usage. In step 712, the intensity score values are, in some embodiments, displayed on a user interface rendered on a visual display device. The intensity score values may be displayed as numbers and/or with the aid of graphical tools, e.g., a graphical display of the scale of intensity scores with current score, and the like. In some embodiments, the intensity score may be indicated by audio. In step 712, the intensity score values are, in some embodiments, displayed along with one or more quantitative or qualitative pieces of information on the user including, but not limited to, whether the user has exceeded his/her anaerobic threshold, the heart rate zones experienced by the user during an exercise routine, how difficult an exercise routine was in the context of the user's training, the user's perceived exertion during an exercise routine, whether the exercise regimen of the user should be automatically adjusted (e.g., made easier if the intensity scores are consistently high), whether the user is likely to experience soreness the next day and the level of expected soreness, characteristics of the exercise routine (e.g., how difficult it was for the user, whether the exercise was in bursts or activity, whether the exercise was tapering, etc.), and the like. In one embodiment, the analytics system may automatically generate, store and display an exercise regimen customized based on the intensity scores of the user.

Step 706 may use any of a number of exemplary static or dynamic weighting schemes that enable the intensity score to be customized and adapted for the unique physiological properties of the user. In one exemplary static weighting scheme, the weights applied to the HRR values are based on static models of a physiological process. The human body employs different sources of energy with varying efficiencies and advantages at different HRR levels. For example, at the anaerobic threshold (AT), the body shifts to anaerobic respiration in which the cells produce two adenosine triphosphate (ATP) molecules per glucose molecule, as opposed to 36 at lower HRR levels. At even higher HRR levels, there is a further subsequent threshold (CPT) at which creatine triphosphate (CTP) is employed for respiration with even less efficiency.

In order to account for the differing levels of cardiovascular exertion and efficiency at the different HRR levels, in one embodiment, the possible values of HRR are divided into a plurality of categories, sections or levels (e.g., three) dependent on the efficiency of cellular respiration at the respective categories. The HRR parameter range may be divided in any suitable manner, such as, piecewise, including piecewise-linear, piecewise-exponential, and the like. An exemplary piecewise-linear division of the HRR parameter range enables weighting each category with strictly increasing values. This scheme captures an accurate indication of the cardiovascular intensity experienced by the user because it is more difficult to spend time at higher HRR values, which suggests that the weighting function should increase at the increasing weight categories.

In one non-limiting example, the HRR parameter range may be considered a range from zero (0) to one (1) and divided into categories with strictly increasing weights. In one example, the HRR parameter range may be divided into a first category of a zero HRR value and may assign this category a weight of zero; a second category of HRR values falling between zero (0) and the user's anaerobic threshold (AT) and may assign this category a weight of one (1); a third category of HRR values falling between the user's anaerobic threshold (AT) and a threshold at which the user's body employs creatine triphosphate for respiration (CPT) and may assign this category a weight of 18; and a fourth category of HRR values falling between the creatine triphosphate threshold (CPT) and one (1) and may assign this category a weight of 42, although other numbers of HRR categories and different weight values are possible. That is, in this example, the weights are defined as:

$$w(v) = \begin{cases} 0 & : v = 0 \\ 1 & : v \in (0, AT] \\ 18 & : v \in (AT, CPT] \\ 42 & : v \in (CPT, 1] \end{cases}$$

In another exemplary embodiment of the weighting scheme, the HRR time series is weighted iteratively based on the intensity scores determined thus far (e.g., the intensity score accrued thus far) and the path taken by the HRR values to get to the present intensity score. The path may be detected automatically based on the historical HRR values and may indicate, for example, whether the user is performing high intensity interval training (during which the intensity scores are rapidly rising and falling), whether the user is taking long breaks between bursts of exercise (during which the intensity scores are rising after longer periods), and the like. The path may be used to dynamically determine and adjust the weights applied to the HRR values. For example, in the case of high intensity interval training, the weights applied may be higher than in the case of a more traditional exercise routine.

In another exemplary embodiment of the weighting scheme, a predictive approach is used by modeling the weights or coefficients to be the coefficient estimates of a logistic regression model. In this scheme, a training data set is obtained by continuously detecting the heart rate time series and other personal parameters of a group of individuals. The training data set is used to train a machine learning system to predict the cardiovascular intensities experienced by the individuals based on the heart rate and other personal data. The trained system models a regression in which the coefficient estimates correspond to the weights or coefficients of the weighting scheme. In the training phase, user input on perceived exertion and the intensity scores are compared. The learning algorithm also alters the weighs based on the improving or declining health of a user as well as their qualitative feedback. This yields a unique algorithm that incorporates physiology, qualitative feedback, and quantitative data. In determining a weighting scheme for a specific user, the trained machine learning system is run by executing computer-executable instructions encoded on one or more non-transitory computer-readable media, and generates the coefficient estimates which are then used to weight the user's HRR time series.

One of ordinary skill in the art will recognize that two or more aspects of any of the disclosed weighting schemes may be applied separately or in combination in an exemplary method for determining an intensity score.

In one aspect, heart rate zones quantify the intensity of workouts by weighing and comparing different levels of heart activity as percentages of maximum heart rate. Analysis of the amount of time an individual spends training at a certain percentage of his/her MHR may reveal his/her state of physical exertion during a workout. This intensity, developed from the heart rate zone analysis, motion, and activity, may then indicate his/her need for rest and recovery after the workout, e.g., to minimize delayed onset muscle soreness (DOMS) and prepare him/her for further activity. As discussed above, MHR, heart rate zones, time spent above the anaerobic threshold, and HRV in RSA (Respiratory Sinus Arrhythmia) regions-as well as personal information (gender, age, height, weight, etc.) may be utilized in data processing.

A recovery score or indicator provides an accurate indication of the level of recovery of a user's body and health after a period of physical exertion. The human autonomic nervous system controls the involuntary aspects of the body's physiology and is typically subdivided into two branches: parasympathetic (deactivating) and sympathetic (activating). Heart rate variability (HRV), i.e., the fluctuation in inter-heartbeat interval time, is a commonly studied result of the interplay between these two competing branches. Parasympathetic activation reflects inputs from internal organs, causing a decrease in heart rate. Sympathetic activation increases in response to stress, exercise and disease, causing an increase in heart rate. For example, when high intensity exercise takes place, the sympathetic response to the exercise persists long after the completion of the exercise. When high intensity exercise is followed by insufficient recovery, this imbalance lasts typically until the next morning, resulting in a low morning HRV. This result should be taken as a warning sign as it indicates that the parasympathetic system was suppressed throughout the night. While suppressed, normal repair and maintenance processes that ordinarily would occur during sleep were suppressed as well. Suppression of the normal repair and maintenance processes results in an unprepared state for the next day, making subsequent exercise attempts more challenging.

The recovery score is customized and adapted for the unique physiological properties of the user and takes into account, for example, the user's heart rate variability (HRV), resting heart rate, sleep quality and recent physiological strain (indicated, in one example, by the intensity score of the user). In one exemplary embodiment, the recovery score is a weighted combination of the user's heart rate variability (HRV), resting heart rate, sleep quality indicated by a sleep score, and recent strain (indicated, in one example, by the intensity score of the user). In an exemplar, the sleep score combined with performance readiness measures (such as, morning heart rate and morning heart rate variability) provides a complete overview of recovery to the user. By considering sleep and HRV alone or in combination, the user can understand how exercise-ready he/she is each day and to understand how he/she arrived at the exercise-readiness score each day, for example, whether a low exercise-readiness score is a predictor of poor recovery habits or an inappropriate training schedule. This insight aids the user in adjusting his/her daily activities, exercise regimen and sleeping schedule therefore obtain the most out of his/her training.

In some cases, the recovery score may take into account perceived psychological strain experienced by the user. In some cases, perceived psychological strain may be detected from user input via, for example, a questionnaire on a mobile device or web application. In other cases, psychological strain may be determined automatically by detecting changes in sympathetic activation based on one or more parameters including, but not limited to, heart rate variability, heart rate, galvanic skin response, and the like.

With regard to the user's HRV used in determining the recovery score, suitable techniques for analyzing HRV include, but are not limited to, time-domain methods, frequency-domain methods, geometric methods and non-linear methods. In one embodiment, the HRV metric of the root-mean-square of successive differences (RMSSD) of RR intervals is used. The analytics system may consider the magnitude of the differences between 7-day moving averages and 3-day moving averages of these readings for a given day. Other embodiments may use Poincaré Plot analysis or other suitable metrics of HRV.

The recovery score algorithm may take into account RHR along with history of past intensity and recovery scores.

With regard to the user's resting heart rate, moving averages of the resting heart rate are analyzed to determine significant deviations. Consideration of the moving averages is important since day-to-day physiological variation is quite large even in healthy individuals. Therefore, the analytics system may perform a smoothing operation to distinguish changes from normal fluctuations.

Although an inactive condition, sleep is a highly active recovery state during which a major portion of the physiological recovery process takes place. Nonetheless, a small, yet significant, amount of recovery can occur throughout the day by rehydration, macronutrient replacement, lactic acid removal, glycogen re-synthesis, growth hormone production and a limited amount of musculoskeletal repair. In assessing the user's sleep quality, the analytics system generates a sleep score using continuous data collected by an exemplary physiological measurement system regarding the user's heart rate, skin conductivity, ambient temperature and accelerometer/gyroscope data throughout the user's sleep. Collection and use of these four streams of data enable an understanding of sleep previously only accessible through invasive and disruptive over-night laboratory testing. For example, an increase in skin conductivity when ambient temperature is not increasing, the wearer's heart rate is low, and the accelerometer/gyroscope shows little motion, may indicate that the wearer has fallen asleep. The sleep score indicates and is a measure of sleep efficiency (how good the user's sleep was) and sleep duration (if the user had sufficient sleep). Each of these measures is determined by a combination of physiological parameters, personal habits and daily stress/strain (intensity) inputs. The actual data measuring the time spent in various stages of sleep may be combined with the wearer's recent daily history and a longer-term data set describing the wearer's personal habits to assess the level of sleep sufficiency achieved by the user. The sleep score is designed to model sleep quality in the context of sleep duration and history. It thus takes advantage of the continuous monitoring nature of the exemplary physiological measurement systems disclosed herein by considering each sleep period in the context of biologically-determined sleep needs, pattern-determined sleep needs and historically-determined sleep debt.

The recovery and sleep score values are stored on a non-transitory storage medium for retrieval, display and usage. The recovery and/or sleep score values are, in some embodiments, displayed on a user interface rendered on a visual display device. The recovery and/or sleep score values may be displayed as numbers and/or with the aid of graphical tools, e.g., a graphical display of the scale of recovery scores with current score, and the like. In some embodiments, the recovery and/or sleep score may be indicated by audio. The recovery score values are, in some embodiments, displayed along with one or more quantitative or qualitative pieces of information on the user including, but not limited to, whether the user has recovered sufficiently, what level of activity the user is prepared to perform, whether the user is prepared to perform an exercise routine a particular desired intensity, whether the user should rest and the duration of recommended rest, whether the exercise regimen of the user should be automatically adjusted (e.g., made easier if the recovery score is low), and the like. In one embodiment, the analytics system may automatically generate, store and display an exercise regimen customized based on the recovery scores of the user alone or in combination with the intensity scores.

As discussed above, the sleep performance metric may be based on parameters like the number of hours of sleep, sleep onset latency, and the number of sleep disturbances. In this manner, the score may compare a tactical athlete's duration and quality of sleep in relation to the tactical athlete's evolving sleep need (e.g., a number of hours based on recent strain, habitual sleep need, signs of sickness, and sleep debt). By way of example, a soldier may have a dynamically changing need for sleep, and it may be important to consider the total hours of sleep in relation to the amount of sleep that may have been required. By providing an accurate sensor for sleep and sleep performance, an aspect may evaluate sleep in the context of the overall day and lifestyle of a specific user.

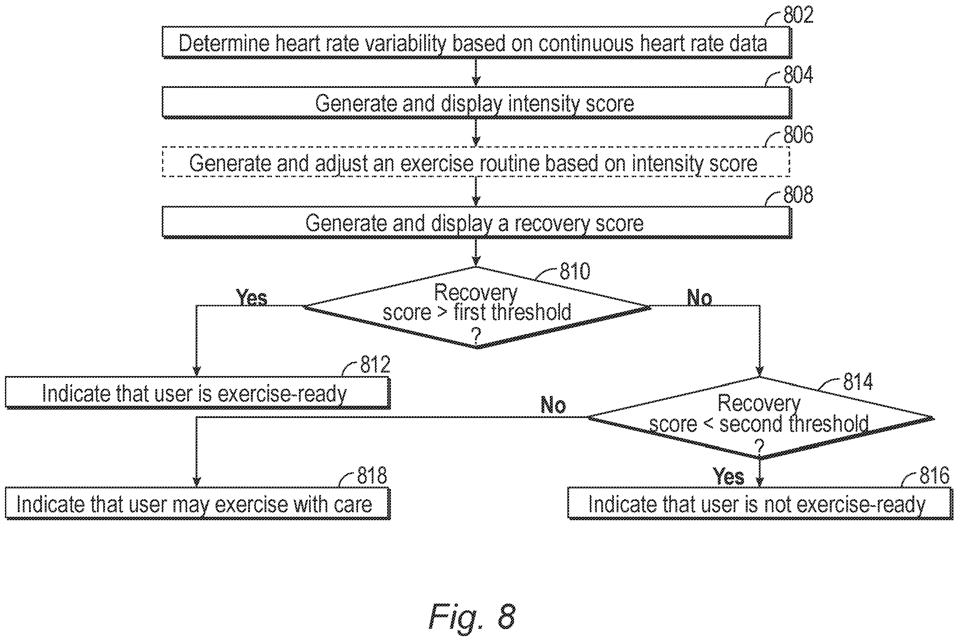

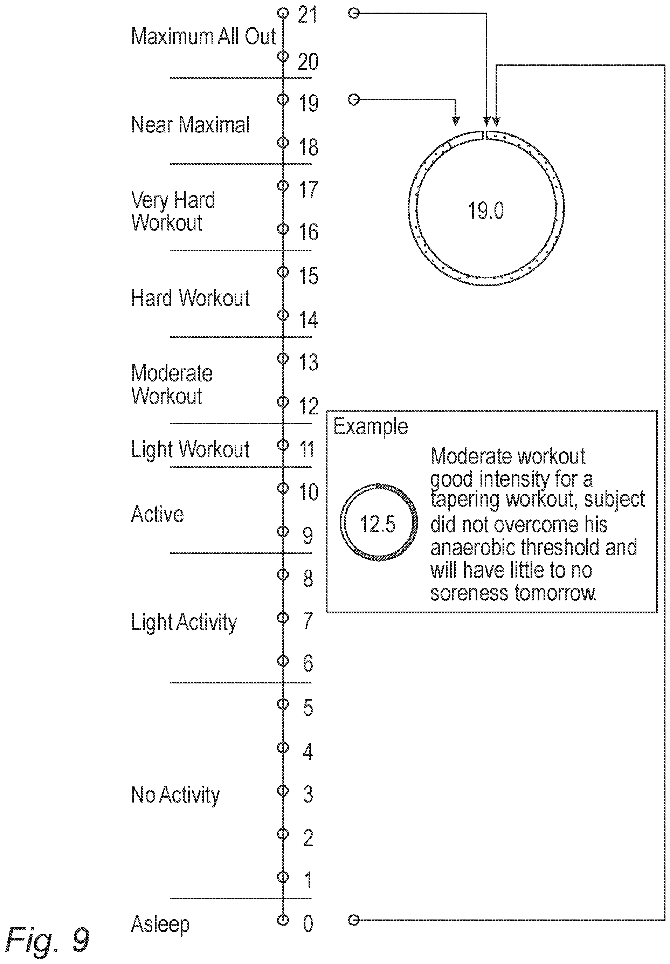

Fig. 8 is a flow chart illustrating an exemplary method by which a user may use intensity and recovery scores. In step 802, the wearable physiological measurement system begins determining heart rate variability (HRV) measurements based on continuous heart rate data collected by an exemplary physiological measurement system. In some cases, it may take the collection of several days of heart rate data to obtain an accurate baseline for the HRV. In step 804, the analytics system may generate and display intensity score for an entire day or an exercise routine. In some cases, the analytics system may display quantitative and/or qualitative information corresponding to the intensity score. Fig. 9 illustrates an exemplary display of an intensity score index indicated in a circular graphic component with an exemplary current score of 19.0 indicated. The graphic component may indicate a degree of difficulty of the exercise corresponding to the current score selected from, for example, maximum all out, near maximal, very hard, hard, moderate, light, active, light active, no activity, asleep, and the like. The display may indicate, for example, that the intensity score corresponds to a good and tapering exercise routine, that the user did not overcome his anaerobic threshold and that the user will have little to no soreness the next day.

In step 806, in an exemplary embodiment, the analytics system may automatically generate or adjust an exercise routine or regimen based on the user's actual intensity scores or desired intensity scores. For example, based on inputs of the user's actual intensity scores, a desired intensity score (that is higher than the actual intensity scores) and a first exercise routine currently performed by the user (e.g., walking), the analytics system may recommend a second different exercise routine that is typically associated with higher intensity scores than the first exercise routine (e.g., running).

In step 808, at any given time during the day (e.g., every morning), the analytics system may generate and display a recovery score. In some cases, the analytics system may display quantitative and/or qualitative information corresponding to the intensity score. For example, in step 810, in an exemplary embodiment, the analytics system may determine if the recovery is greater than (or equal to or greater than) a first predetermined threshold (e.g., about 60% to about 80% in some examples) that indicates that the user is recovered and is ready for exercise. If this is the case, in step 812, the analytics system may indicate that the user is ready to perform an exercise routine at a desired intensity or that the user is ready to perform an exercise routine more challenging than the past day's routine. Otherwise, in step 814, the analytics system may determine if the recovery is lower than (or equal to or lower than) a second predetermined threshold (e.g., about 10% to about 40% in some examples) that indicates that the user has not recovered. If this is the case, in step 816, the analytics system may indicate that the user should not exercise and should rest for an extended period. The analytics system may, in some cases, the duration of recommended rest. Otherwise, in step 818, the analytics system may indicate that the user may exercise according to his/her exercise regimen while being careful not to overexert him/herself. The thresholds may, in some cases, be adjusted based on a desired intensity at which the user desires to exercise. For example, the thresholds may be increased for higher planned intensity scores.

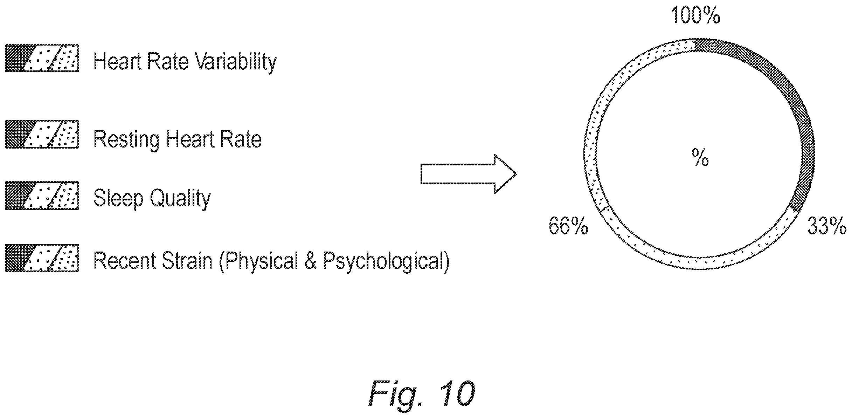

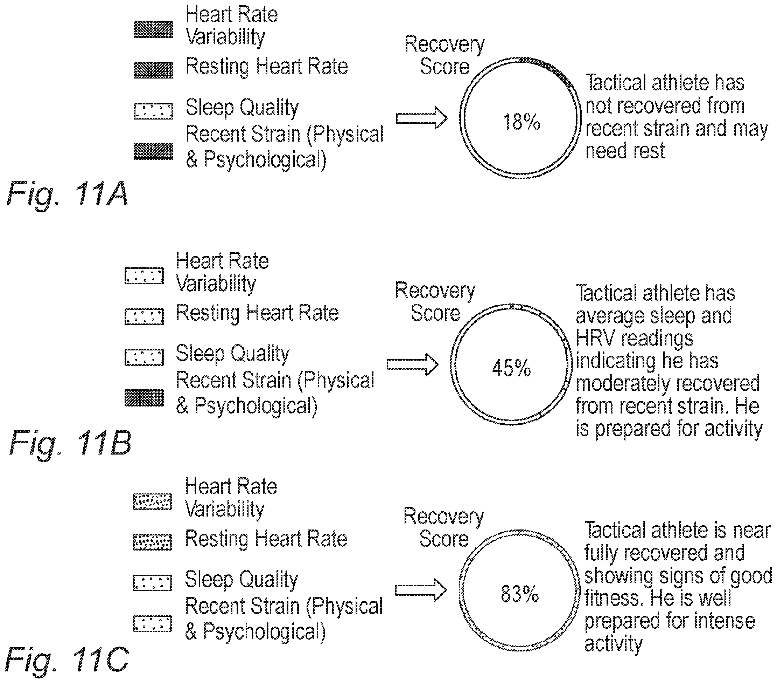

Fig. 10 illustrates an exemplary display of a recovery score index indicated in a circular graphic component with a first threshold of 66% and a second threshold of 33% indicated. Figs. 11A-11C illustrate the recovery score graphic component with exemplary recovery scores and qualitative information corresponding to the recovery scores.

Optionally, in an exemplary embodiment, the analytics system may automatically generate or adjust an exercise routine or regimen based on the user's actual recovery scores (e.g., to recommend lighter exercise for days during which the user has not recovered sufficiently). This process may also use a combination of the intensity and recovery scores.

The analytics system may, in some embodiments, determine and display the intensity and/or recovery scores of a plurality of users in a comparative manner. This enables users to match exercise routines with others based on comparisons among their intensity scores.

III. Exemplary Computing Devices

Various aspects and functions described herein may be implemented as hardware, software or a combination of hardware and software on one or more computer systems. Exemplary computer systems that may be used include, but are not limited to, personal computers, embedded computing systems, network appliances, workstations, mainframes, networked clients, servers, media servers, application servers, database servers, web servers, virtual servers, and the like. Other examples of computer systems that may be used include, but are not limited to, mobile computing devices, such as wearable devices, cellular phones and personal digital assistants, and network equipment, such as load balancers, routers and switches.

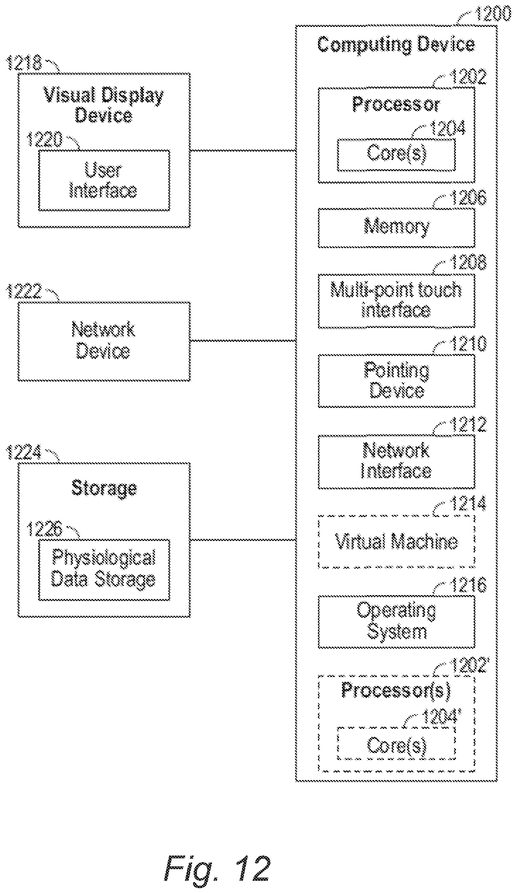

Fig. 12 is a block diagram of an exemplary computing device 1200 that may be used in to perform any of the methods provided by exemplary embodiments. The computing device may be configured as an embedded system in the integrated circuit board(s) of a wearable physiological measurements system and/or as an external computing device that may receive data from a wearable physiological measurement system.

The computing device 1200 includes one or more non-transitory computer-readable media for storing one or more computer-executable instructions or software for implementing exemplary embodiments. The non-transitory computer-readable media may include, but are not limited to, one or more types of hardware memory, non-transitory tangible media (for example, one or more magnetic storage disks, one or more optical disks, one or more USB flash drives), and the like. For example, memory 1206 included in the computing device 1200 may store computer-readable and computer-executable instructions or software for implementing exemplary embodiments. The computing device 1200 also includes processor 1202 and associated core 1204, and optionally, one or more additional processor(s) 1202' and associated core(s) 1204' (for example, in the case of computer systems having multiple processors/cores), for executing computer-readable and computer-executable instructions or software stored in the memory 1206 and other programs for controlling system hardware. Processor 1202 and processor(s) 1202' may each be a single core processor or multiple core (1204 and 1204') processor.

Virtualization may be employed in the computing device 1200 so that infrastructure and resources in the computing device may be shared dynamically. A virtual machine 1214 may be provided to handle a process running on multiple processors so that the process appears to be using only one computing resource rather than multiple computing resources. Multiple virtual machines may also be used with one processor.

Memory 1206 may include a computer system memory or random access memory, such as DRAM, SRAM, EDO RAM, and the like. Memory 1206 may include other types of memory as well, or combinations thereof.

A user may interact with the computing device 1200 through a visual display device 1218, such as a computer monitor, which may display one or more user interfaces 1220 that may be provided in accordance with exemplary embodiments. The visual display device 1218 may also display other aspects, elements and/or information or data associated with exemplary embodiments, for example, views of databases, photos, and the like. The computing device 1200 may include other I/O devices for receiving input from a user, for example, a keyboard or any suitable multi-point touch interface 1208, a pointing device 1210 (e.g., a mouse). The keyboard 1208 and the pointing device 1210 may be coupled to the visual display device 1218. The computing device 1200 may include other suitable conventional I/O peripherals.

The computing device 1200 may also include one or more storage devices 1224, such as a hard-drive, CD-ROM, or other computer readable media, for storing data and computer-readable instructions and/or software that implement exemplary methods as taught herein. Exemplary storage device 1224 may also store one or more databases 1226 for storing any suitable information required to implement exemplary embodiments. The databases may be updated by a user or automatically at any suitable time to add, delete or update one or more items in the databases.

The computing device 1200 may include a network interface 1212 configured to interface via one or more network devices 1222 with one or more networks, for example, Local Area Network (LAN), Wide Area Network (WAN) or the Internet through a variety of connections including, but not limited to, standard telephone lines, LAN or WAN links (for example, 802.11, T1, T3, 56kb, X.25), broadband connections (for example, ISDN, Frame Relay, ATM), wireless connections, controller area network (CAN), or some combination of any or all of the above. The network interface 1212 may include a built-in network adapter, network interface card, PCMCIA network card, card bus network adapter, wireless network adapter, USB network adapter, modem or any other device suitable for interfacing the computing device 1200 to any type of network capable of communication and performing the operations described herein. Moreover, the computing device 1200 may be any computer system, such as a workstation, desktop computer, server, laptop, handheld computer, tablet computer (e.g., the iPad® tablet computer), mobile computing or communication device (e.g., the iPhone® communication device), or other form of computing or telecommunications device that is capable of communication and that has sufficient processor power and memory capacity to perform the operations described herein.

The wearable physiological measurement system may record and transmit at least the following types of data to an external computing system, mobile communication system or the Internet: raw continuously-detected data (e.g., heart rate data, movement data, galvanic skin response data) and processed data based on the raw data (e.g., RR intervals determined from the heart rate data). Transmission modes may be wired (e.g., using USB stick inserted into a USB port on the system) or wireless (e.g., using a wireless transmitter). The raw and processed data may be transmitted together or separately using different transmission modes. Since a raw data file is typically substantially larger than a processed data file, the raw data file may be transmitted using WiFi or a USB stick, while the processed data file may be transmitted using Bluetooth.

An exemplary wearable system may include a 2G, 3G or 4G chip that wirelessly uploads all data to the website disclosed herein without requiring any other external device. A 3G or 4G chip may be used preferably as a 2G connection on a Nokia 5800 was found to transfer data at a rate of 520 kbps using 1.69 W, while a 3G connection transferred at 960 kbps using 1.73 W. Therefore, the 3G chip would use negligibly more power for almost twice the transfer speed, thereby halving half the transfer time and using much less energy from the battery.

In some cases, the wearable system may opportunistically transfer data when in close proximity to a streaming outlet. For example, the system may avoid data transmission when it is not within close proximity of a streaming outlet, and, when nearby a streaming outlet (e.g., a linked phone), may send the data to the external device via Bluetooth and to the Internet via the external device. This is both convenient and "free" in the sense that it utilizes existing cellular data plans.

Limiting the frequency with which data is streamed increases the wearable system's battery life. In one non-limiting example, the system may be set to stream automatically in the morning and following a time stamp. Regardless of the data transmission scheme, the system stores all the data it collects. Data may also be streamed on demand by a user, for example, by turning a physical component on the system and holding it or by initiating a process on the mobile application or receiving device. In some embodiments, the data transmission frequency may be automatically adjusted based on one or more physiological parameters, e.g., heart rate. For example, higher heart rates may prompt more frequent and real-time streaming transmission of data.

The computing device 1200 may run any operating system 1216, such as any of the versions of the Microsoft® Windows® operating systems, the different releases of the Unix and Linux operating systems, any version of the MacOS® for Macintosh computers, any embedded operating system, any real-time operating system, any open source operating system, any proprietary operating system, any operating systems for mobile computing devices, or any other operating system capable of running on the computing device and performing the operations described herein. In exemplary embodiments, the operating system 1216 may be run in native mode or emulated mode. In an exemplary embodiment, the operating system 1216 may be run on one or more cloud machine instances.

IV. Exemplary Network Environments

Various aspects and functions of the implementations may be distributed among one or more computer systems configured to provide a service to one or more client computers, or to perform an overall task as part of a distributed system. Additionally, aspects may be performed on a client-server or multi-tier system that includes components distributed among one or more server systems that perform various functions. Thus, the implementations are not limited to executing on any particular system or group of systems. Further, aspects may be implemented in software, hardware or firmware, or any combination thereof. Thus, aspects may be implemented within methods, acts, systems, system placements and components using a variety of hardware and software configurations, and they are not limited to any particular distributed architecture, network or communication protocol. Furthermore, aspects may be implemented as specially-programmed hardware and/or software.

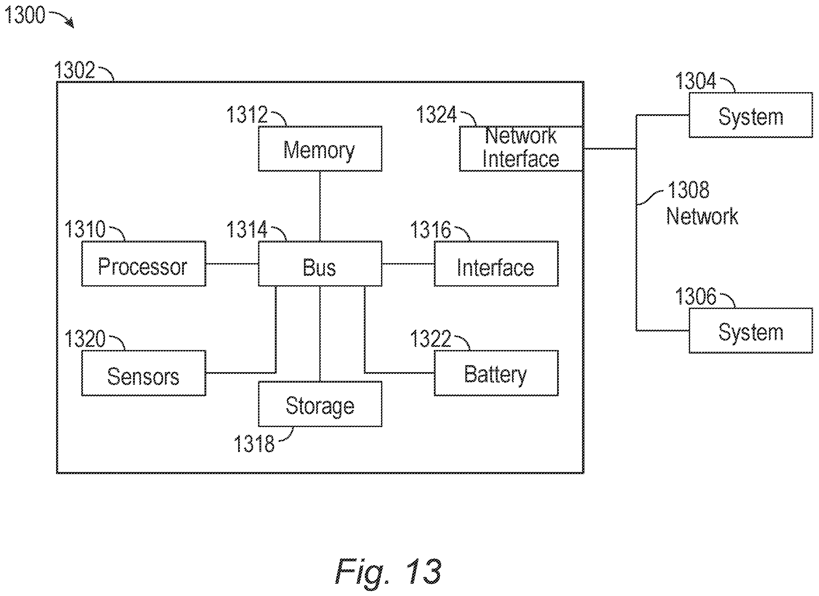

Fig. 13 is a block diagram of an exemplary system 1300, e.g., a distributed computer system in which various aspects and functions may be practiced. The distributed computer system 1300 may include one or more computer systems. For example, as illustrated, the distributed computer system 1300 includes three computer systems 1302, 1304 and 1306. As shown, the computer systems 1302, 1304, 1306 are interconnected by, and may exchange data through, a communication network 1308. The network 1308 may include any communication network through which computer systems may exchange data. To exchange data via the network 1308, the computer systems and the network may use various methods, protocols and standards including, but not limited to, token ring, Ethernet, wireless Ethernet, Bluetooth, TCP/IP, UDP, HTTP, FTP, SNMP, SMS, MMS, SS7, JSON, XML, REST, SOAP, CORBA, IIOP, RMI, DCOM and Web Services. To ensure data transfer is secure, the computer systems may transmit data via the network using a variety of security measures including, but not limited to, TSL, SSL and VPN. While the distributed computer system 1300 illustrates three networked computer systems, the distributed computer system may include any number of computer systems, networked using any medium and communication protocol.

Various aspects and functions may be implemented as specialized hardware or software executing in one or more computer systems. As depicted, the computer system 1300 includes a processor 1310, a memory 1312, a bus 1314, an interface 1316 and a storage system 1318. The processor 1310, which may include one or more microprocessors or other types of controllers, can perform a series of instructions that manipulate data. The processor 1310 may be a well-known commercially-available processor such as an Intel Pentium, Intel Atom, ARM Processor, Motorola PowerPC, SGI MIPS, Sun UltraSPARC or Hewlett-Packard PA-RISC processor, or may be any other type of processor or controller as many other processors and controllers are available. The processor 1310 may be a mobile device or smart phone processor, such as an ARM Cortex processor, a Qualcomm Snapdragon processor or an Apple processor. As shown, the processor 1310 is connected to other system placements, including a memory 1312, by the bus 1314.

The memory 1312 may be used for storing programs and data during operation of the computer system 1300. Thus, the memory 1312 may be a relatively high performance, volatile, random access memory such as a dynamic random access memory (DRAM) or static memory (SRAM). However, the memory 1312 may include any device for storing data, such a disk drive or other non-volatile storage device, such as flash memory or phase-change memory (PCM). Various embodiments can organize the memory 1312 into particularized and, in some cases, unique structures to perform the aspects and functions disclosed herein.

Components of the computer system 1300 may be coupled by an interconnection element such as the bus 1314. The bus 1314 may include one or more physical busses (for example, buses between components that are integrated within the same machine) and may include any communication coupling between system placements including specialized or standard computing bus technologies such as IDE, SCSI, PCI and InfiniBand. Thus, the bus 1314 enables communications (for example, data and instructions) to be exchanged between system components of the computer system 1300.

Computer system 1300 also includes one or more interface devices 1316, such as input devices, output devices and combination input/output devices. The interface devices 1316 may receive input, provide output, or both. For example, output devices may render information for external presentation. Input devices may accept information from external sources. Examples of interface devices include, but are not limited to, keyboards, mouse devices, trackballs, microphones, touch screens, printing devices, display screens, speakers, network interface cards, and the like. The interface devices 1316 allow the computer system 1300 to exchange information and communicate with external entities, such as users and other systems.

Storage system 1318 may include one or more computer-readable and computer-writeable non-volatile and non-transitory storage media on which computer-executable instructions are encoded that define a program to be executed by the processor. The storage system 1318 also may include information that is recorded on or in the media, and this information may be processed by the program. More specifically, the information may be stored in one or more data structures specifically configured to conserve storage space or increase data exchange performance. The instructions may be persistently stored as encoded signals, and the instructions may cause a processor to perform any of the functions described herein. A medium that can be used with various embodiments may include, for example, optical disk, magnetic disk or flash memory, among others. In operation, the processor 1310 or some other controller may cause data to be read from the non-transitory recording media into another memory, such as the memory 1312, that allows for faster access to the information by the processor than does the storage medium included in the storage system 1318. The memory may be located in the storage system 1318 and/or in the memory 1312. The processor 1310 may manipulate the data within the memory 1312, and then copy the data to the medium associated with the storage system 1318 after processing is completed. A variety of components may manage data movement between the media and the memory 1312, and the present disclosure is not limited thereto.

Further, the implementations are not limited to a particular memory system or storage system. Although the computer system 1300 is shown by way of example as one type of computer system upon which various aspects and functions may be practiced, aspects are not limited to being implemented on the computer system. Various aspects and functions may be practiced on one or more computers having different architectures or components than that shown in the illustrative figures. For instance, the computer system 1300 may include specially-programmed, special-purpose hardware, such as for example, an application-specific integrated circuit (ASIC) tailored to perform a particular operation disclosed herein. Another embodiment may perform the same function using several general-purpose computing devices running MAC OS® System X with Motorola PowerPC® processors and several specialized computing devices running proprietary hardware and operating systems.

The computer system 1300 may include an operating system that manages at least a portion of the hardware placements included in computer system 1300. A processor or controller, such as processor 1310, may execute an operating system which may be, among others, a Windows-based operating system (for example, Windows NT, Windows 2000/ME, Windows XP, Windows 7, or Windows Vista) available from the Microsoft Corporation, a MAC OS® System X operating system available from Apple Computer, one of many Linux-based operating system distributions (for example, the Enterprise Linux operating system available from Red Hat Inc.), a Solaris operating system available from Sun Microsystems, or a UNIX operating systems available from various sources. The operating system may be a mobile device or smart phone operating system, such as Windows Mobile, Android or iOS. Many other operating systems may be used, and embodiments are not limited to any particular operating system.

The processor and operating system together define a computing platform for which application programs in high-level programming languages may be written. These component applications may be executable, intermediate (for example, C# or JAVA bytecode) or interpreted code which communicate over a communication network (for example, the Internet) using a communication protocol (for example, TCP/IP). Similarly, functions may be implemented using an object-oriented programming language, such as SmallTalk, JAVA, C++, Ada, or C# (C-Sharp). Other object-oriented programming languages may also be used. Alternatively, procedural, scripting, or logical programming languages may be used.

Additionally, various functions may be implemented in a non-programmed environment (for example, documents created in HTML, XML or other format that, when viewed in a window of a browser program, render aspects of a graphical-user interface or perform other functions). Further, various embodiments may be implemented as programmed or non-programmed placements, or any combination thereof. For example, a web page may be implemented using HTML while a data object called from within the web page may be written in C++. Thus, the implementations are not limited to a specific programming language and any suitable programming language could also be used.

A computer system included within an embodiment may perform functions outside the scope of the embodiment. For instance, aspects of the system may be implemented using an existing product. Aspects of the system may be implemented on database management systems such as SQL Server available from Microsoft of Seattle, Washington; Oracle Database from Oracle of Redwood Shores, California; and MySQL from Sun Microsystems of Santa Clara, California; or integration software such as WebSphere middleware from IBM of Armonk, New York. However, a computer system running, for example, SQL Server may be able to support both aspects in accord with the implementations and databases for sundry applications not within the scope of the disclosure.

It is also understood that a system 1302 of the distributed computer system 1300 may include a wearable physiological measurement system, e.g., configured to provide collection and monitoring of physiological data. In this manner, the system 1302 may include one or more sensors 1320. As discussed herein, the sensors 1320 may include a heart rate monitor. In some embodiments, the system 1302 may further include one or more of sensors 1320 for detecting calorie burn, distance, and activity. Calorie burn may be based on a user's heart rate, and a calorie burn measurement may be improved if a user chooses to provide his or her weight and/or other physical parameters. In some embodiments, manual entering of data is not required in order to derive calorie burn; however, data entry may be used to improve the accuracy of the results. In some embodiments, if a user has forgotten to enter a new weight, he/she can enter it for past weeks and the calorie burn may be updated accordingly.

The sensors 1320 may include one or more sensors for activity measurement. In some embodiments, the system 1302 may include one or more multi-axes accelerometers and/or gyroscope to provide a measurement of activity. In some embodiments, the accelerometer may further be used to filter a signal from the optical sensor for measuring heart rate and to provide a more accurate measurement of the heart rate. In some embodiments, the system 1302 may include a multi-axis accelerometer to measure motion and calculate distance, whether it be in real terms as steps or miles or as a converted number. Activity sensors may be used, for example, to classify or categorize activity, such as walking, running, performing another sport, standing, sitting or lying down. In some embodiments, one or more of collected physiological data may be aggregated to generate an aggregate activity level. For example, heart rate, calorie burn, and distance may be used to derive an aggregate activity level. The aggregate level may be compared with or evaluated relative to previous recordings of the user's aggregate activity level, as well as the aggregate activity levels of other users.

The sensors 1320 may include a thermometer for monitoring the user's body or skin temperature. In one embodiment, the sensors 1320 may be used to recognize sleep based on a temperature drop, GSR data, lack of activity according to data collected by the accelerometer, and reduced heart rate as measured by the heart rate monitor. The body temperature, in conjunction with heart rate monitoring and motion, may be used to interpret whether a user is sleeping or just resting, as body temperature drops significantly when an individual is about to fall asleep), and how well an individual is sleeping as motion indicates a lower quality of sleep. The body temperature may also be used to determine whether the user is exercising and to categorize and/or analyze activities.

The system 1302 may further include one or more batteries 1322. According to one embodiment, the one or more batteries 1322 may be configured to allow continuous wear and usage of the system 1302. In one embodiment, the system 1302 may include two or more batteries 1322. The system 1302 may include a removable battery that may be recharged using a charger. In one example, the removable battery may be configured to slip in and out of a head portion of the system, attach onto the bracelet, or the like. In one example, the removable battery may be able to power the system for around a week. Additionally, the system 1302 may include a built-in battery. The built-in battery may be recharged by the removable battery. The built-in battery may be configured to power the bracelet for around a day on its own. When the more removable battery is being charged, the user does not need to remove the system 1302 and may continue collecting data using the built-in battery. In other embodiments, the two batteries 1322 may both be removable and rechargeable.

In some embodiments, the system 1302 may include a battery 1322 that is a wireless rechargeable battery. For example, the battery 1322 may be recharged by placing the system or the battery on a rechargeable mat. In other example, the battery 1322 may be a long range wireless rechargeable battery. In other embodiments, the battery 1322 may be a rechargeable via motion. In yet other embodiments, the battery 1322 may be rechargeable using a solar energy source.

The wearable system 1302 may include one or more non-transitory computer-readable media (the storage 1318) for storing raw data detected by the sensors 1320 of the system 1302 and processed data calculated by the processor 1310 of the system. Thus, this system 1302 may include a processor 1310, a memory (the storage 1318), a bus 1314, a network interface 1324, and an interface 1316. The network interface 1324 may be configured to wirelessly communicate data to an external network. Some embodiments of the wearable system 1302 may be configured to stream information wirelessly to a social network. In some embodiments, data streamed from a user's wearable system to an external network may be accessed by the user via a website. The network interface 1324 may be configured such that data collected by the system 1302 may be streamed wirelessly. In some embodiments, data may be transmitted automatically, without the need to manually press any buttons. In some embodiments, the system may include a cellular chip built into the system 1302. In one example, the network interface 1324 may be configured to stream data using Bluetooth technology. In another example, the network interface 1324 may be configured to stream data using a cellular data service, such as via a 3G, 4G, or 5G cellular network.

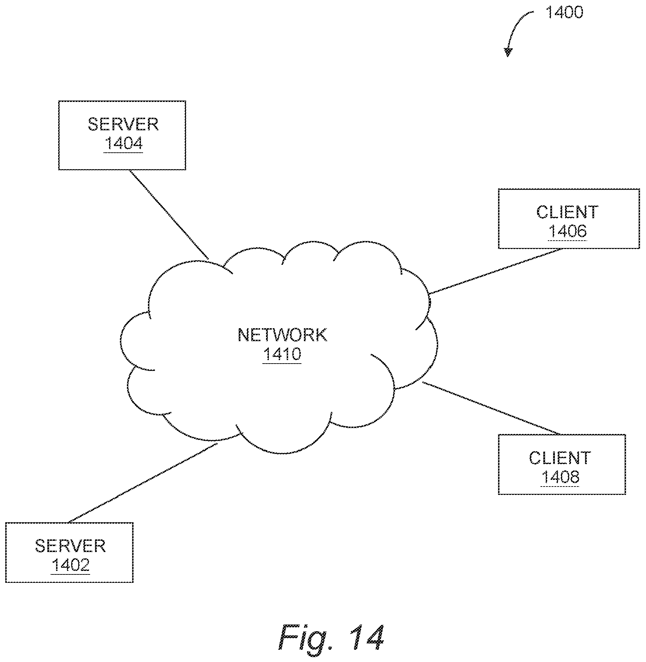

Fig. 14 is a diagram of an exemplary network environment 1400 suitable for a distributed implementation of exemplary embodiments. The network environment 1400 may include one or more servers 1402 and 1404 coupled to one or more clients 1406 and 1408 via a communication network 1410. The network interface 1212 and the network device 1222 of the computing device 1200 enable the servers 1402 and 1404 to communicate with the clients 1406 and 1408 via the communication network 1410. The communication network 1410 may include, but is not limited to, the Internet, an intranet, a LAN (Local Area Network), a WAN (Wide Area Network), a MAN (Metropolitan Area Network), a wireless network, an optical network, and the like. The communication facilities provided by the communication network 1410 are capable of supporting distributed implementations of exemplary embodiments.

In an exemplary embodiment, the servers 1402 and 1404 may provide the clients 1406 and 1408 with computer-readable and/or computer-executable components or products under a particular condition, such as a license agreement. For example, the computer-readable and/or computer-executable components or products may include those for providing and rendering any of the user interfaces disclosed herein. The clients 1406 and 1408 may provide and render an exemplary graphical user interface using the computer-readable and/or computer-executable components and products provided by the servers 1402 and 1404.

Alternatively, in another exemplary embodiment, the clients 1406 and 1408 may provide the servers 1402 and 1404 with computer-readable and computer-executable components or products under a particular condition, such as a license agreement. For example, in an exemplary embodiment, the servers 1402 and 1404 may provide and render an exemplary graphical user interface using the computer-readable and/or computer-executable components and products provided by the clients 1406 and 1408.

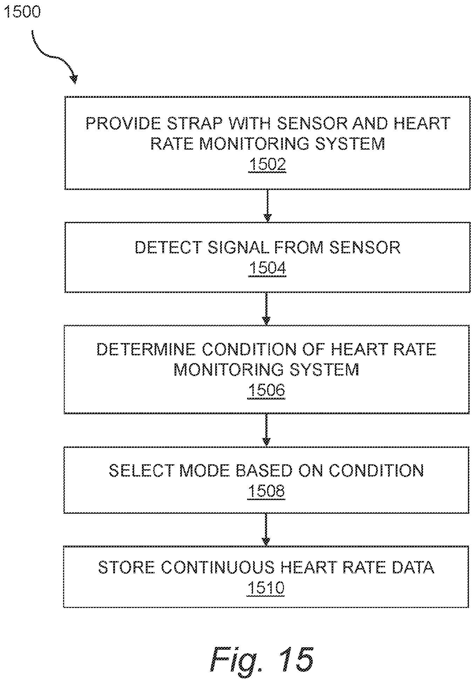

Fig. 15 is a flow chart illustrating a method for selecting modes of acquiring heart rate data.

As shown in step 1502, the method 1500 may include providing a strap with a sensor and a heart rate monitoring system. The strap may be shaped and sized to fit about an appendage. For example, the strap may be any of the straps described herein, including, without limitation, a bracelet. The heart rate monitoring system may be configured to provide two or more different modes for detecting a heart rate of a wearer of the strap. The modes may include the use of optical detectors (e.g., light detectors), light emitters, motion sensors, a processing module, algorithms, other sensors, a peak detection technique, a frequency domain technique, variable optical characteristics, non-optical techniques, and so on.

As shown in step 1504, the method 1500 may include detecting a signal from the sensor. The signal may be detected by one or more sensors, which may include any of the sensors described herein. The signal may include, without limitation, one or more signals associated with the heart rate of the user, other physiological signals, an optical signal, signals based on movement, signals based on environmental factors, status signals (e.g., battery life), historical information, and so on.

As shown in step 1506, the method 1500 may include determining a condition of the heart rate monitoring system, which may be based upon the signal. The condition may include, without limitation, an accuracy of heart rate detection determined using a statistical analysis to provide a confidence level in the accuracy, a power consumption, a battery charge level, a user activity, a location of the sensor or motion of the sensor, an environmental or contextual condition (e.g., ambient light conditions), a physiological condition, an active condition, an inactive condition, and so on. This may include detecting a change in the condition, responsively selecting a different one of the two or more different modes, and storing additional continuous heart rate data obtained using at least one of the two or more different modes.

As shown in step 2508, the method 1500 may include selecting one of the two or more different modes for detecting the heart rate based on the condition. For example, based on the motion status of the user, the method may automatically and selectively activate one or more light emitters to determine a heart rate of the user. The system may also or instead determine the type of sensor to use at a given time based on the level of motion, skin temperature, heart rate, and the like. Based on a combination of these factors the system may selectively choose which type of sensor to use in monitoring the heart rate of the user. A processor or the like may be configured to select one of the modes. For example, if the condition is the accuracy of heart rate detection determined using a statistical analysis to provide a confidence level in the accuracy, the processor may be configured to select a different one of the modes when the confidence level is below a predetermined threshold.

As shown in step 1510, the method 1500 may include storing continuous heart rate data using one of the two or more different modes. This may include communicating the continuous heart rate data from the strap to a remote data repository. This may also or instead include storing the data locally, e.g., on a memory included on the strap. The memory may be removable, e.g., via a data card or the like, or the memory may be permanently attached/integral with the strap or a component thereof. The stored data (e.g., heart rate data) may be for the user's private use, for example, when in a private setting, or the data may be shared when in a shared setting (e.g., on a social networking site or the like). The method 1500 may further include the use of a privacy switch operable by the user to controllably restrict communication of a portion of the data, e.g., to the remote data repository.

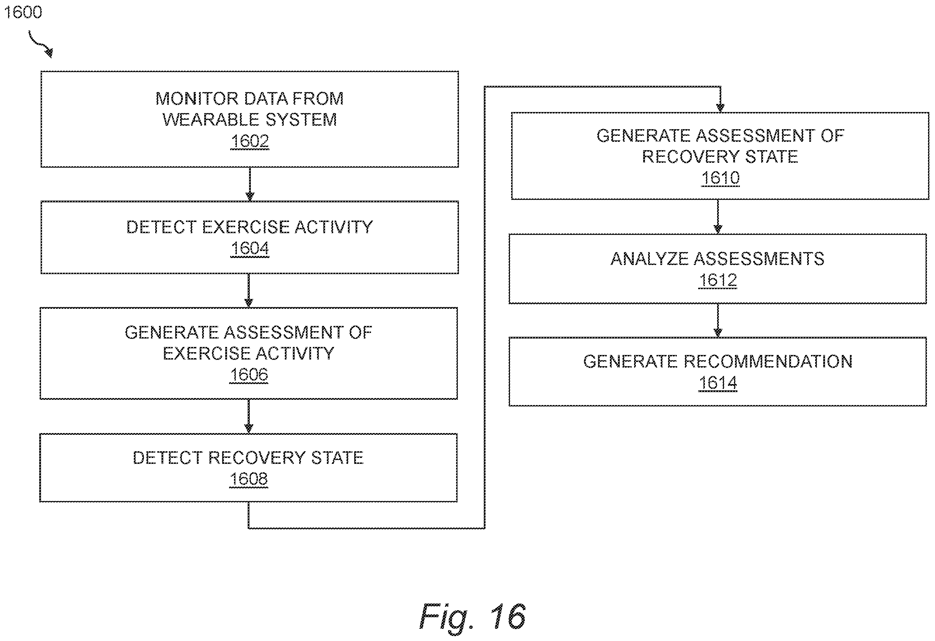

Fig. 16 is a flow chart of a method for assessing recovery and making exercise recommendations.

As shown in step 1602, the method 1600 may include monitoring data from a wearable system. The wearable system may be a continuous-monitoring, physiological measurement system worn by a user. The data may include heart rate data, other physiological data, summary data, motion data, fitness data, activity data, or any other data described herein or otherwise contemplated by a skilled artisan.

As shown in step 1604, the method 1600 may include detecting exercise activity. This may include automatically detecting exercise activity of the user. The exercise activity may be detected through the use of one or more sensors as described herein. The exercise activity may be sent to a server that, e.g., performs step 1606 described below.

As shown in step 1606, the method 1600 may include generating an assessment of the exercise activity. This may include generating a quantitative assessment of the exercise activity. Generating a quantitative assessment of the exercise activity may include analyzing the exercise activity on a remote server. Generating a quantitative assessment may include the use of the algorithms discussed herein. The method 1600 may also include generating periodic updates to the user concerning the exercise activity. The method 1600 may also include determining a qualitative assessment of the exercise activity and communicating the qualitative assessment to the user.

As shown in step 1608, the method 1600 may include detecting a recovery state. This may include automatically detecting a physical recovery state of the user. The recovery state may be detected through the use of one or more sensors as described herein. The recovery state may be sent to a server that, e.g., performs step 1610 described below.

As shown in step 1610, the method 1600 may include generating an assessment of the recovery state. This may include generating a quantitative assessment of the physical recovery state. Generating a quantitative assessment may include the use of the algorithms discussed herein. Generating a quantitative assessment of the physical recovery state may include analyzing the physical recovery state on a remote server. The method 1600 may also include generating periodic updates to the user concerning the physical recover state. The method 1600 may also include determining a qualitative assessment of the recovery state and communicating the qualitative assessment to the user.

As shown in step 1612, the method 1600 may include analyzing the assessments, i.e., analyzing the quantitative assessment of the exercise activity and the quantitative assessment of the physical recovery. The analysis may include the use of one or more of the algorithms described herein, a statistical analysis, and so on. The analysis may include the use of a remote server.

As shown in step 1614, the method 1600 may include generating a recommendation. This may include automatically generating a recommendation on a change to an exercise routine of the user based on the analysis performed in step 1612. This may also or instead include determining a qualitative assessment of the exercise activity and/or recovery state, and communicating the qualitative assessment(s) to the user. The recommendation may be generated on a remote server. The recommendation may be communicated to the user in an electronic mail, it may be presented to the user in a web page, other communications interface, or the like. Generating the recommendation may be based upon a number of cycles of exercise and rest.

The method 1600 described above, or any of the methods discussed herein, may also or instead be implemented on a computer program product including non-transitory computer executable code embodied in a non-transitory computer-readable medium that executes on one or more computing devices to perform the method steps. For example, code may be provided that performs the various steps of the methods described herein.

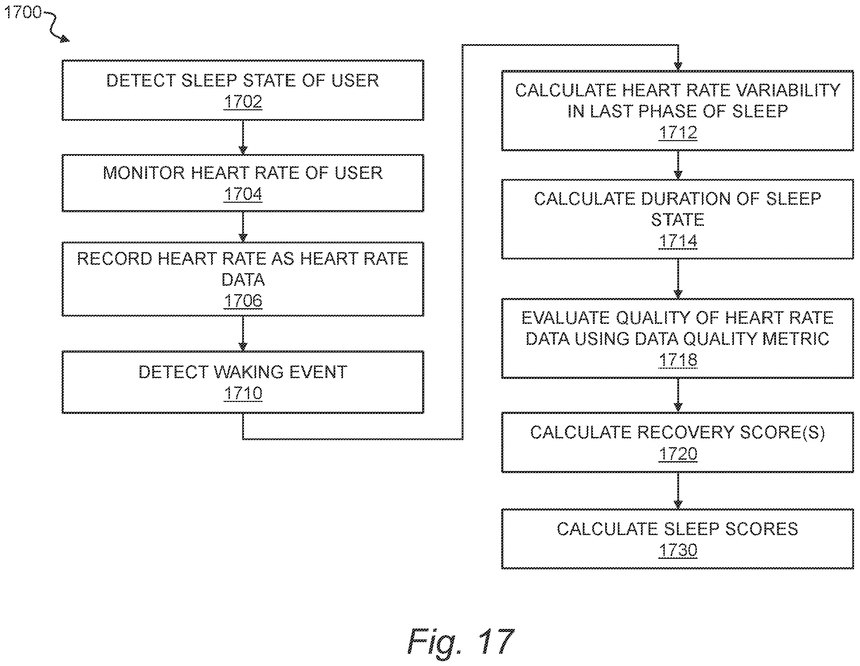

Fig. 17 is a flow chart illustrating a method for detecting heart rate variability in sleep states. The method 1700 may be used in cooperation with any of the devices, systems, and methods described herein, such as by operating a wearable, continuous physiological monitoring device to perform the following steps. The wearable, continuous physiological monitoring system may for example include a processor, one or more light emitting diodes, one or more light detectors configured to obtain heart rate data from a user, and one or more other sensors to assist in detecting stages of sleep. In general, the method 1700 aims to measure heart rate variability in the last phase of sleep before waking in order to provide a consistent and accurate basis for calculating a physical recovery score.

As shown in step 1702, the method 1700 may include detecting a sleep state of a user. This may, for example, include any form of continuous or periodic monitoring of sleep states using any of a variety of sensors or algorithms as generally described herein.

Sleep states (also be referred to as "sleep phases," "sleep cycles," "sleep stages," or the like) may include rapid eye movement (REM) sleep, non-REM sleep, or any states/stages included therein. The sleep states may include different phases of non-REM sleep, including Stages 1-3. Stage 1 of non-REM sleep generally includes a state where a person's eyes are closed, but the person can be easily awakened; Stage 2 of non-REM sleep generally includes a state where a person is in light sleep, i.e., where the person's heart rate slows and their body temperature drops in preparation for deeper sleep; and Stage 3 of non-REM sleep generally includes a state of deep sleep, where a person is not easily awakened. Stage 3 is often referred to as delta sleep, deep sleep, or slow wave sleep (i.e., from the high amplitude but small frequency brain waves typically found in this stage). Slow wave sleep is thought to be the most restful form of sleep, which relieves subjective feelings of sleepiness and restores the body.

REM sleep on the other hand typically occurs 1-2 hours after falling asleep. REM sleep may include different periods, stages, or phases, all of which may be included within the sleep states that are detected as described herein. During REM sleep, breathing may become more rapid, irregular and shallow, eyes may jerk rapidly (thus the term "Rapid Eye Movement" or "REM"), and limb muscles may be temporarily paralyzed. Brain waves during this stage typically increase to levels experienced when a person is awake. Also, heart rate, cardiac pressure, cardiac output, and arterial pressure may become irregular when the body moves into REM sleep. This is the sleep state in which most dreams occur, and, if awoken during REM sleep, a person can typically remember the dreams. Most people experience three to five intervals of REM sleep each night.

Homeostasis is the balance between sleeping and waking, and having proper homeostasis may be beneficial to a person's health. Lack of sleep is commonly referred to as sleep deprivation, which tends to cause slower brain waves, a shorter attention span, heightened anxiety, impaired memory, mood disorders, and general mental, emotional, and physical fatigue. Sleep debt (the effect of not getting enough sleep) may result in the diminished abilities to perform high-level cognitive functions. A person's circadian rhythms (i.e., biological processes that display an endogenous, entrainable oscillation of about 24 hours) may be a factor in a person's optimal amount of sleep. Thus, sleep may in general be usefully monitored as a proxy for physical recovery. However, a person's heart rate variability at a particular moment during sleep - during the last phase of sleep preceding a waking event -- can further provide an accurate and consistent basis for objectively calculating a recovery score following a period of sleep.

According to the foregoing, sleep of a user may be monitored to detect various sleep states, transitions, and other sleep-related information. For example, the device may monitor/detect the duration of sleep states, the transitions between sleep states, the number of sleep cycles or particular states, the number of transitions, the number of waking events, the transitions to an awake state, and so forth. Sleep states may be monitored and detected using a variety of strategies and sensor configurations according to the underlying physiological phenomena. For example, body temperature may be usefully correlated to various sleep states and transitions. Similarly, galvanic skin response may be correlated to sweating activity and various sleep states, any of which may also be monitored, e.g., with a galvanic skin response sensor, to determine sleep states. Physical motion can also be easily monitored using accelerometers or the like, which can be used to detect waking or other activity involving physical motion. In another aspect, heart rate activity itself may be used to infer various sleep states and transitions, either alone or in combination with other sensor data. Other sensors may also or instead be used to monitor sleep activity, such as brain wave monitors, pupil monitors, and so forth, although the ability to incorporate these types of detection into a continuously wearable physiological monitoring device may be somewhat limited depending on the contemplated configuration.

As shown in step 1704, the method 1700 may include monitoring a heart rate of the user substantially continuously with the continuous physiological monitoring system. Continuous heart rate monitoring is described above in significant detail, and the description is not repeated here except to note generally that this may include raw sensor data, heart rate data or peak data, and heart rate variability data over some historical period that can be subsequently correlated to various sleep states and activities.

As shown in step 1706, the method 1700 may include recording the heart rate as heart rate data. This may include storing the heart rate data in any raw or processed form on the device, or transmitting the data to a local or remote location for storage. In one aspect, the data may be stored as peak-to-peak data or in some other semi-processed form without calculating heart rate variability. This may be useful as a technique for conserving processing resources in a variety of contexts, for example where only the heart rate variability at a particular time is of interest. Data may be logged in some unprocessed or semi-processed form, and then the heart rate variability at a particular point in time can be calculated once the relevant point in time has been identified.

As shown in step 1710, the method 1700 may include detecting a waking event at a transition from the sleep state of the user to an awake state. It should be appreciated that the waking event may be a result of a natural termination of sleep, e.g., after a full night's rest, or in response to an external stimulus that causes awakening prior to completion of a natural sleep cycle. Regardless of the precipitating event(s), the waking event may be detected via the various physiological changes described above, or using any other suitable techniques. While the emphasis herein is on a wearable, continuous monitoring device, it will be understood that the device may also receive inputs from an external device such as a camera (for motion detection) or an infrared camera (for body temperature detection) that can be used to aid in accurately assessing various sleep states and transitions.

Thus the wearable, continuous physiological monitoring system may generally detect a waking event using one or more sensors including, for example, one or more of an accelerometer, a galvanic skin response sensor, a light sensor, and so forth. For example, in one aspect, the waking event may be detected using a combination of motion data and heart rate data.

As shown in step 1712, the method 1700 may include calculating a heart rate variability of the user at a moment in a last phase of sleep preceding the waking event based upon the heart rate data. While a waking event and a history of sleep states are helpful information for assessing recovery, the method 1700 described herein specifically contemplates use of the heart rate variability in a last phase of sleep as a consistent foundation for calculating recovery scores for a device user. Thus step 1712 may also include detecting a slow wave sleep period immediately prior to the waking event, or otherwise determining the end of a slow wave or deep sleep episode immediately preceding the waking event.

It will be appreciated that the last phase of sleep preceding a natural waking event may be slow wave sleep. However, where a sleeper is awakened prematurely, this may instead include a last recorded episode of REM sleep or some other phase of sleep immediately preceding the waking event. This moment - the end of the last phase of sleep before waking -- is the point at which heart rate variability data provides the most accurate and consistent indicator of physical recovery. Thus, with the appropriate point of time identified, the historical heart rate data (in whatever form) may be used with the techniques described above to calculate the corresponding heart rate variability. It will be further noted that the time period for this calculation may be selected with varying degrees of granularity depending on the ability to accurate detect the last phase of sleep and an end of the last phase of sleep. Thus for example, the time may be a predetermined amount of time before waking, or at the end of slow wave sleep, or some predetermined amount of time before the end of slow wave sleep is either detected or inferred. In another aspect, an average heart rate variability or similar metric may be determined for any number of discrete measurements within a window around the time of interest.

As shown in step 1714, the method 1700 may include calculating a duration of the sleep state. The quantity and quality of sleep may be highly relevant to physical recovery, and as such the duration of the sleep state may be used to calculate a recovery score.

As shown in step 1718, the method 1700 may include evaluating a quality of heart rate data using a data quality metric for a slow wave sleep period, e.g., the slow wave sleep period occurring most recently before the waking event. As noted above, the quality of heart rate measurements may vary over time for a variety of reasons. Thus the quality of heart rate data may be evaluated prior to selecting a particular moment or window of heart rate data for calculating heart rate variability, and the method 1700 may include using this quality data to select suitable values for calculating a recovery score. For example, the method 1700 may include calculating the heart rate variability for a window of predetermined duration within the slow wave sleep period having the highest quality of heart rate data according to the data quality metric.

As shown in step 1720, the method 1700 may include calculating a recovery score for the user based upon the heart rate variability from the last phase of sleep. The calculation may be based on other sources of data. For example, the calculation of recovery score may be based on the duration of sleep, the stages of sleep detected or information concerning the stages (e.g., amount of time in certain stages), information regarding the most recent slow wave sleep period or another sleep period/state, information from the GSR sensor or other sensor(s), and so on. The method 1700 may further include calculating additional recovery scores after one or more other waking events of the user for comparison to the previously calculated recovery score. The actual calculation of a discovery score is described in substantial detail above, and this description is not repeated here except to note that the use of a heart rate variability measurement from the last phase of sleep provides an accurate and consistent basis for evaluating the physical recovery state of a user following a period of sleep.

As shown in step 1730, the method 1700 may include calculating a sleep score and communicating this score to a user.

In one aspect, the sleep score may be a measure of prior sleep performance. For example, a sleep performance score may quantify, on a scale of 0-100, the ratio of the hours of sleep during a particular resting period compared to the sleep needed. On this scale, if a user sleeps six hours and needed eight hours of sleep, then the sleep performance may be calculated as 75%. The sleep performance score may begin with one or more assumptions about needed sleep, based on, e.g., age, gender, health, fitness level, habits, genetics, and so forth and may be adapted to actual sleep patterns measured for an individual over time.

The sleep score may also or instead include a sleep need score or other objective metric that estimates an amount of sleep needed by the user of the device in a next sleep period. In general, the score may be any suitable quantitative representation including, e.g., a numerical value over some predetermined scale (e.g., 0-10, 1-100, or any other suitable scale) or a representation of a number of hours of sleep that should be targeted by the user. In another aspect, the sleep score may be calculated as the number of additional hours of sleep needed beyond a normal amount of sleep for the user.

The score may be calculated using any suitable inputs that capture, e.g., a current sleep deficit, a measure of strain or exercise intensity over some predetermined prior interval, an accounting for any naps or other resting, and so forth. A variety of factors may affect the actual sleep need, including physiological attributes such as age, gender, health, genetics and so forth, as well as daytime activities, stress, napping, sleep deficit or deprivation, and so forth. The sleep deficit may itself be based on prior sleep need and actual sleep performance (quality, duration, waking intervals, etc.) over some historical window. In one aspect, an objective scoring function for sleep need may have a model of the form:

$$SleepNeed = Baseline + f_1(strain) + f_2(debt) - Naps$$

In general, this calculation aims to estimate the ideal amount of sleep for best rest and recovery during a next sleep period. When accounting for time falling asleep, periods of brief wakefulness, and so forth, the actual time that should be dedicated to sleep may be somewhat higher, and this may be explicitly incorporated into the sleep need calculation, or left for a user to appropriately manage sleep habits.

In general, the baseline sleep may represent a standard amount of sleep needed by the user on a typical rest day (e.g., with no strenuous exercise or workout). As noted above, this may depend on a variety of factors, and may be estimated or measured for a particular individual in any suitable manner. The strain component, fl(strain), may be assessed based on a previous day's physical intensity, and will typically increase the sleep need. Where intensity or strain is measured on an objective scale from 0 to 21, the strain calculation may take the following form, which yields an additional sleep time needed in minutes for a strain, i:

$$f(i) = \frac{1.7}{1 + e^{\frac{17-i}{3.5}}}$$

The sleep debt, $f_2(debt)$, may generally measure a carryover of needed sleep that was not attained in a previous day. This may be scaled, and may be capped at a maximum, according to individual sleep characteristics or general information about long term sleep deficit and recovery. Naps may also be accounted for directly by correcting the sleep need for any naps that have been taken, or by calculating a nap factor that is scaled or otherwise manipulated or calculated to more accurately track the actual effect of naps on prospective sleep need.

However calculated, the sleep need may be communicated to a user, such as by displaying a sleep need on a wrist-worn physiological monitoring device, or by sending an e-mail, text message or other alert to the user for display on any suitable device.

VI. Infection Monitoring

Infection monitoring may be accomplished using physiological monitoring systems and techniques as described herein, e.g., where data is provided by sensors disposed on a wearable physiological monitoring device such as any described herein.

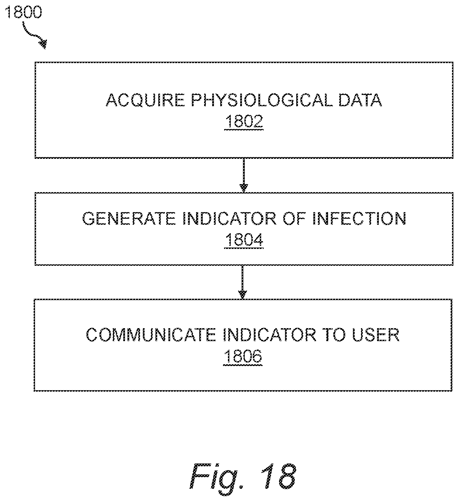

Fig. 18 is a flow chart of a method for creating an indicator of a physiological condition. In general, this may include determining a baseline respiratory rate for a user based on historical data from a wearable physiological monitoring device, and comparing this baseline to a current interval of interest. This general technique may be used, for example, for early detection of the onset of a condition such as a respiratory infection or other illness that can be correlated to physiological signals captured by the wearable physiological monitoring device.

In general, the method 1800 may include acquiring a physiological data for a user (also referred to herein as a "wearer"), such as a physiological data signal from a wearable device worn by the user over a period of time including a recent window (for which an evaluation is to be performed) and a historical window (upon which a baseline is established) preceding the recent window. The physiological data signal may, for example, include heart rate data acquired using photoplethysmography, any other continuous heart rate data, or the like. The physiological data may also or instead include acoustic data, accelerometer data, or other data that might be used to detect or infer a respiratory rate for a wearer of the device, or to augment or refine calculations of a physiological signal using other data sources. The physiological data signal may directly measure respiratory rate or a respiratory rate pattern, and/or the physiological data signal may provide a proxy for a respiratory pattern of the user. In one aspect, variations in heart rate may be correlated to inhalation and exhalation, and used to infer a corresponding respiratory rate. It will be understood that acquiring the physiological data signal may include transmitting the physiological data signal to a remote server for processing. The physiological data signal may also or instead be processed on the wearable device, and/or preprocessed on the device before transmitting as a processed physiological data signal to the server. A continuous physiological monitor may advantageously be employed in this context to facilitate post hoc identification of relevant activity periods and processing of corresponding intervals of physiological data.

For measurements such as respiratory rate, these measurements may usefully be taken during windows of sleep, e.g., during particular sleep stages where the respiratory rate is typically relatively highly consistent for healthy individuals. Thus, for example, a recent window may include a sleep interval such as a most recent sleep interval, or a most recent sleep interval of a particular stage of sleep. Similarly, the historical window may include one or more previous intervals of sleep, e.g., from previous cycles of the same stage of sleep used for the recent interval. This may, for example, include measurements from a consistent period such as a period of deep sleep most immediately preceding the end of a nightly sleep event, or most immediately following the beginning of the nightly sleep. Sleep intervals may be detected by the wearable device, e.g., according to sleep metrics described elsewhere herein. Within such a window, any of a variety of metrics may be used to estimate or calculate respiratory rate. For example, an average respiratory rate for the entire stage of sleep may be used, or an average or median respiratory rate for some sub-interval of the stage of sleep may be used, such as the last minute prior to an end of the stage of sleep. This may also or instead account for a natural end of a stage of sleep, e.g., by excluding from the baseline a stage of sleep that appears to have terminated prematurely by a waking event. Similarly, a current interval may be measured based on a most recent, available window for a stage of sleep that has concluded naturally rather than due to an interruption.

In other aspects, the quality of heart rate data may be used to select a suitable window, and/or various respiratory rates may be measured over some historical period for the wearer, and a time for measuring the rate for an individual may be selected based on when, for that individual, the respiratory rate is most consistent or otherwise would be beneficial for use in the present teachings. More generally, any suitable windowing technique for capturing a substantially consistent respiratory rate data for a user may be used to establish the historical window for comparison as contemplated herein. In this manner, the historical window of one or more prior intervals of sleep may be used to establish a baseline, healthy respiratory heart rate for comparison to new measurements. The number of measurements or intervals in the historical window used to establish the respiratory baseline may be a fixed number of measurements based on an analysis of respiratory rate data for a larger population, or the number may be selected for an individual based on, e.g., how quickly measurements appear to converge on a respiratory rate with sufficiently small variability to serve as a baseline for subsequent comparisons. More generally, the historical window may include any interval or group of intervals sufficient to establish a pre-infection baseline for a health respiratory pattern.

In general, a recent window used for comparison to the baseline may be captured using the same timing techniques that was used for the historical window that established the baseline respiratory heart rate, e.g., from the same moment within a sleep stage or sleep interval that was used for the historical window that established the baseline, healthy respiratory heart rate. The comparison may be any comparison suitable for detecting variations consistent with a presence of an infection or the onset of infection symptoms. For example, this may include a simple quantitative comparison of the current respiratory rate-in this context, the "current" rate refers to the last respiratory rate measured under baseline conditions as described herein-to a mean baseline rate. However, this comparison is also or instead be amenable to classification using machine learning. Thus, in one aspect, the method 1800 may include training a machine classifier to return a probability that a set of one or more features is indicative of an infection, and applying the machine classifier to these features of the physiological data signal during the recent window. It will also be understood that the respiratory rate may be characterized using features in any useful form, including without limitation as a scalar, as a feature vector, or some combination of these, and/or in any other manner suitable for capturing characteristics of the respiratory rate suitable for detecting infection as described herein. In one aspect, once the classification model is created, the model may be deployed on a wearable physiological monitor.

It will be understood that the term "features" as used herein, refers to the metric being measured. Thus, different intervals of time may be characterized with the same "feature," although the "feature" (or feature vector(s) or the like) may have different values over different intervals. It is the differences in each feature that provide a basis for comparison of a current interval to one or more historical intervals that form a baseline.

As noted above, sleep intervals may provide a useful window for substantially consistent measurements of respiratory rate. A variety of techniques are known for detecting the beginning and end of sleep, as well as for distinguishing among different stages of sleep (e.g., slow wave sleep, light sleep, REM sleep, etc.), using data from a wearable physiological monitor such as photoplethysmography or other heart rate data, body temperature data, galvanic skin response data, acoustic data, motion data, and so forth. These or any other techniques to detect sleep-related activity including the onset or end of sleep, as well as transitions among different stages of sleep, may be used to determine suitable moments or intervals for measuring respiratory rate as contemplated herein.

As shown in step 1804, the method 1800 may include generating an indicator of infection for the user based on the data. In general, the indicator may be for an infection or other illness, a likelihood of an infection or other illness, or any other indicator that might usefully be derived from physiological monitoring and provided to a user. The indicator may, for example, be a daily indicator calculated at least once per day and presented to the user at a suitable time, such as when the user arises from sleep. Where the likelihood of infection is evaluated in whole or in part based on, e.g., data over a sleep interval for the user or other daily patterns, the likelihood will typically be evaluated and presented once per day. However, an indicator may also or instead be continuously evaluated and presented to the user, or presented on some other schedule or interval. In one aspect, the indicator may be conditionally presented to the user, for example, only when the likelihood of infection is above some predetermined threshold.

In one aspect, the indicator may be calculated by a server that receives physiological data from a wearable physiological monitor. Alternatively, where a suitable machine learning model or the like can be deployed on the wearable physiological monitor, or on some other local user device, the indicator may be locally evaluated and presented to the user without intervention or assistance by a server or other remote resource.

As shown in step 1806, the method 1800 may include communicating the indicator, and/or information related thereto, to the user. That is, the indicator may be transmitted to a device associated with the user (e.g., for display) such as a laptop computer, a tablet, a cellular phone, or the like. For example, the device may include a user interface such as a display, an audio output, one or more lights, or the like suitable for presenting the indicator directly to the user through the wearable physiological monitoring device. In another aspect, the indicator may be generated on the wearable device and transmitted locally to another user device such as a cellular phone, laptop computer, desktop computer, tablet, or the like, and/or transmitted to a remote server where the indicator may be stored, further processed, and/or communicated to other user devices for display. In an aspect, the method 1800 includes automatically generating the indicator on the wearable device and transmitting a daily indicator to a device associated with the user.

The indicator may include a score or the like. Such a score may coincide with the potential for the wearer having an infection or other condition, and could be presented with, or could otherwise include, other metrics such as HRV, RHR, and so on. The infection may, for example, be a respiratory infection such as a Covid-19 infection from the SARS-CoV-2 virus. The infection may also or instead include other respiratory infections or conditions detected through changes in a respiratory rate of the user, or any other infection or the like that can be reliably detected based on changes in the physiological signals from the wearable physiological monitoring device.

The indicator may be presented to a user in a variety of formats. For example, the indicator may provide a binary indictor (e.g., "healthy" v. "infected"), a written or qualitative indicator (e.g., "high likelihood of infection," "moderate likelihood of infection," "low likelihood of infection," "negligible likelihood of infection."). In another aspect, the indicator may present a quantitative indicator such as a percentage or other statistical measure of a likelihood of infection. The indicator may also or instead be color coded for presentation to the user, e.g., using green for low/no likelihood of infection, yellow for a moderate likelihood of infection, and red for a high likelihood of infection.

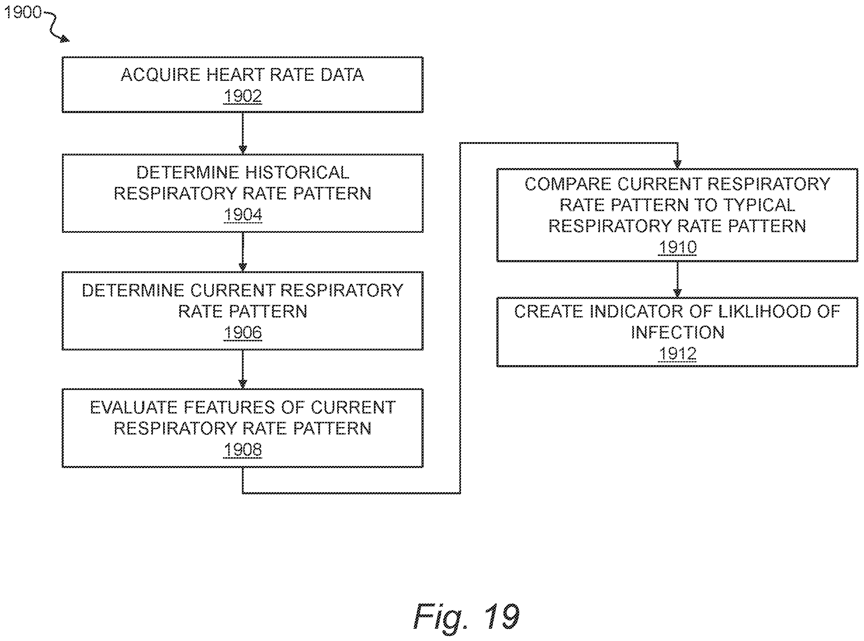

Fig. 19 is a flow chart of a method of infection monitoring. The method 1900 may be implemented with one or more of the devices and systems described herein-e.g., a wearable physiological monitoring system and device. In general, the method 1900 may be used to detect a variation in a physiological attribute of from a baseline or typical threshold that has been established through analysis of historical data from a substantially continuous physiological monitor. By way of example, the method 1900 may be used to detect a variation in a respiratory rate from a respiratory rate baseline that can then be used as an early indicator of the onset of certain infectious conditions such as Covid-19.

As shown in step 1902, the method 1900 may include acquiring heart rate data from a user with a wearable physiological monitor. As discussed above, the wearable physiological monitor may be any as described herein, such as a wearable bracelet or the like that the user wears all or most of the time thereby enabling substantially continuous physiological monitoring. Moreover, the heart rate data that is collected by the wearable physiological monitor may be any as described herein, and for example, may include photoplethysmography (PPG) data or heart rate variability (HRV), which may be derived from the PPG data, e.g., based on peak-to-peak intervals in a heart rate signal in the PPG data.

As shown in step 1904, the method 1900 may include determining a historical respiratory rate pattern for the user based on the heart rate data. The historical respiratory rate pattern may be determined at any suitable intervals. For example, the historical respiratory rate may be analyzed in daily intervals, and may be based on a window within such daily intervals such as a window during sleep, or within a stage of sleep. The historical respiratory rate pattern may characterize one or more features of a typical respiratory rate pattern during these intervals. Thus, the typical or historical respiratory rate pattern may be based on a respiratory rate at a certain time interval within the day for the user, and may be used as a comparative baseline for a current or most recently measured respiratory rate at the corresponding time interval within the day. This historical respiratory rate patten may thus establish a baseline for the user-where respiratory rate patterns can be compared to this baseline for determining whether the user is experiencing a condition of interest, such as whether the user has a respiratory infection or the like. It will be noted that one or more features of the historical respiratory rate pattern may be used as a comparative basis for the same one or more features of the current respiratory rate pattern. For example, this may include a mean respiratory rate over some window, a median respiratory rate over some window, a variance or standard deviation for the respiratory rate over some window, or any other suitable statistical measure. Other features may also or instead be used, such as a maximum rate, minimum rate, range of rates, rate of change in the respiratory rate, and so forth, as well as combinations of the foregoing. Where appropriate for a particular physiological signal, other descriptive mathematical measures may also or instead be used. For example, the slope of a rise in a measured heart signal or a peak-to-trough magnitude may be used to describe the physiological signal and may be used as a basis for comparison to historical norms.

As shown in step 1906, the method 1900 may include determining a current respiratory rate pattern for the user during an interval. The interval may be any useful interval such as any of the predetermined daily intervals described above. The respiratory rate pattern and an underlying respiratory rate may be derived from heart rate data, or otherwise measured or inferred. As described herein, a number of predetermined daily intervals used to establish a baseline and/or a second predetermined daily interval that is being analyzed for variation from the baseline may include sleep intervals detected using data from the wearable physiological monitor. Sleep intervals may advantageously be used to provide a substantially consistent measurement period for respiratory rates with little variation from day to day for a healthy user. By taking respiratory rate measurements at a consistent physiological time, e.g., within a window during a particular stage of sleep, or still more specifically, during the last instance of deep sleep or slow wave sleep before waking, a consistent basis for comparison to the historical baseline can be provided, resulting in improved sensitivity to anomalous patterns and better detection of actual or possible respiratory infections.

As shown in step 1908, the method 1900 may include evaluating one or more features of the current respiratory rate pattern. This may include any useful calculation or other determination of descriptive statistical or mathematical features of the respiratory rate including any of the features described herein such as a mean, median, standard deviation, rate of change, and so forth. In another aspect, this may include providing raw respiratory rate data, or features extracted therefrom, to a trained machine learning algorithm for detection of anomalies indicative of a likelihood of infection, in which case step 1908 and step 1910 may be combined into a single processing step.

As shown in step 1910, the method 1900 may include comparing one or more features of the current respiratory rate pattern to one or more features of the typical or historical respiratory rate pattern. Comparing these features may be performed on a remote server, e.g., where the remote server deploys a machine learning model or the like. Also or instead, comparing these features may be performed by a machine learning model or the like deployed at least in part on the wearable physiological monitor to support detection locally using physiological data directly on the device. In one aspect, the historical respiratory rate pattern may be measured for a particular user wearing the monitoring device. In another aspect, the historical respiratory rate pattern may be based on a population of users, such as an entire population or random selection of users, or a population of users selected for similarity (e.g., in demographics, fitness level, and so forth) to the current user. The historical respiratory rate pattern may also or instead include a combination of individual data for the user and population data for set of other users (which may or may not include the current user).

As shown in step 1912, the method 1900 may include, in response to a predetermined difference between one or more features of the current respiratory rate pattern and one or more features of the typical respiratory rate pattern, creating an indicator of a likelihood of a condition of interest. In certain aspects, the condition of interest is whether the user has a Covid-19 infection or the like, however the method 1900 may also or instead determine a likelihood of respiratory infection generally, or any other condition or combination of conditions that can be detected based on variations in physiological data from an historical baseline. The indicator may be sent to a device associated with the user, e.g., a laptop computer, a tablet, a cellular phone and/or smartphone associated with the user, the wearable physiological monitor itself, another computing device, and the like.

It will be understood that the method 1900 described above, similar to other methods and techniques described herein, may be implemented using a computer program product having computer executable code embodied in a non-transitory computer readable medium that, when executing on one or more computing devices, performs one or more of the steps of the method 1900. For example, execution may be performed on a single device or distributed among a wearable physiological monitor, a cell phone or other user device controlled by the user, and/or a remote server or other processing resource.

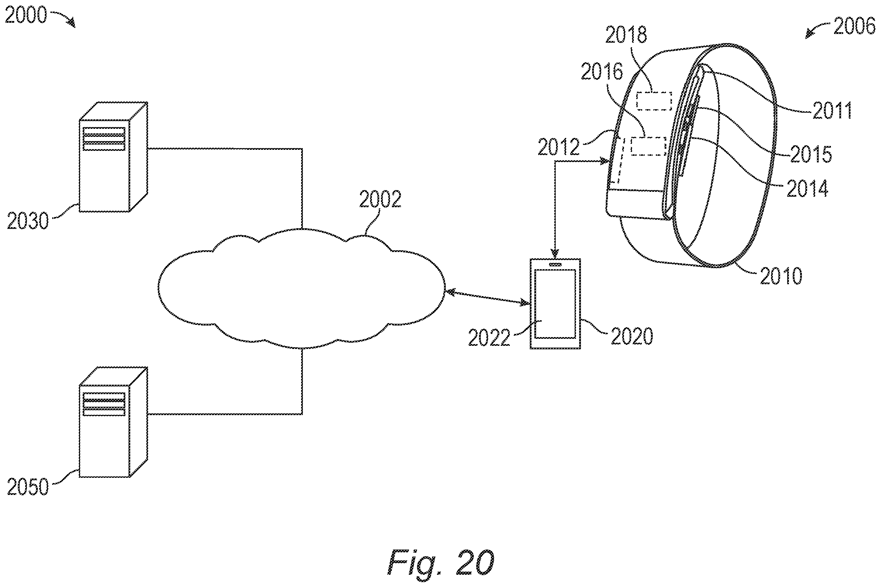

Fig. 20 illustrates a physiological monitoring system. More specifically, Fig. 20 illustrates a physiological monitoring system 2000 that may be used with any of the methods or devices described herein, such as the infection monitoring devices and methods. In general, the system 2000 may include a physiological monitor 2006, a user device 2020, a remote server 2030 with a remote data processing resource (such as any of the processors or processing resources described herein), and one or more other resources 2050, all of which may be interconnected through a data network 2002.

The data network 2002 may be any of the data networks described herein. For example, the data network 2002 may be any network(s) or internetwork(s) suitable for communicating data and information among participants in the system 2000. This may include public networks such as the Internet, private networks, telecommunications networks such as the Public Switched Telephone Network or cellular networks using third generation (e.g., 3G or IMT-2000), fourth generation (e.g., LTE (E-UTRA) or WiMAX-Advanced (IEEE 802.16m)), fifth generation (e.g., 5G), and/or other technologies, as well as any of a variety of corporate area or local area networks and other switches, routers, hubs, gateways, and the like that might be used to carry data among participants in the system 2000. This may also include local or short range communications networks suitable, e.g., for coupling the physiological monitor 2006 to the user device 2020, or otherwise communicating with local resources.

The physiological monitor 2006 may, in general, be any physiological monitoring device, such as any of the wearable monitors or other monitoring devices described herein. Thus, the physiological monitor 2006 may generally be shaped and sized to be worn on a wrist or other appendage of a user and retained in a desired orientation relative to the appendage with a strap 2010 or other attachment mechanism. The physiological monitor 2006 may include a wearable housing 2011, a network interface 2012, one or more sensors 2014, one or more light sources 2015, a processor 2016, a memory 2018, and a wearable strap 2010 for retaining the physiological monitor 2006 in a desired location on a user.

In general, the physiological monitor 2006 may include a wearable physiological monitor configured to acquire heart rate data and/or other physiological data from a wearer. More specifically, the wearable housing 2011 of the physiological monitor 2006 may be configured such that a user can wear a wearable physiological monitor 2006 configured to acquire heart rate data and/or other physiological data from the user in a substantially continuous manner. The wearable housing 2011 may be configured for cooperation with a strap 2010 or the like, e.g., for engagement with an appendage of a user.

The network interface 2012 may be configured to coupled one or more participants of the system 2000 in a communicating relationship, e.g., with the remote server 2030.

The one or more sensors 2014 may include any of the sensors described herein, or any other sensors suitable for physiological monitoring. By way of example and not limitation, the one or more sensors 2014 may include one or more of a light source, and optical sensor, an accelerometer, a gyroscope, a temperature sensor, a galvanic skin response sensor, an environmental sensor (e.g., for measuring ambient temperature, humidity, lighting, and the like), a geolocation sensor, a temporal sensor, an electrodermal activity sensor, and the like. The one or more sensors 2014 may be disposed in the wearable housing 2011, or otherwise positioned and configured for capture of data for physiological monitoring of a user. In one aspect, the one or more sensors 2014 include a light detector configured to provide data to the processor 2018 for calculating a heart rate variability. The one or more sensors 2014 may also or instead include an accelerometer configured to provide data to the processor 2018, e.g., for detecting a sleep state, a waking event, exercise, and/or other user activity. In an implementation, the one or more sensors 2014 measure a galvanic skin response of the user.

The processor 2016 and memory 2018 may be any of the processors and memories described herein, and may be suitable for deployment in a physiological monitoring device. In one aspect, the memory 2018 may store physiological data obtained by monitoring a user with the one or more sensors 2014. The processor 2016 may be configured to obtain heart rate data from the user based on the data from the sensors 2014. The processor 2016 may be further configured to assist in a determination of a condition of the user, such as whether the user has an infection or other condition of interest as described herein.

The one or more light sources 2015 may be coupled to the wearable housing 2011 and controlled by the processor 2018. At least one of the light sources 2015 may be directed toward the skin of a user's appendage. Light from the light source 2015 may be detected by the one or more sensors 2014.

The system 2000 may further include a remote data processing resource executing on a remote server 2030. The remote data processing resource may be any of the processors described herein, and may be configured to receive data transmitted from the memory 2018 of the physiological monitor 2006, and to evaluate a condition of the user such as whether the user has an infection or other condition of interest as described herein.

The system 2000 may also include one or more user devices 2020, which may work together with the physiological monitor 2006, e.g., to provide a display for user data and analysis, and/or to provide a communications bridge from the network interface 2012 of the physiological monitor 2006 to the data network 2002 and the remote server 2030. For example, physiological monitor 2006 may communicate locally with a user device 2020, such as a smartphone of a user, via short-range communications, e.g., Bluetooth, or the like, e.g., for the exchange of data between the physiological monitor 2006 and the user device 2020, and the user device 2020 may communicate with the remote server 2030 via the data network 2002. Computationally intensive processing, such as infection monitoring, may be performed at the remote server 2030, which may have greater memory capabilities and processing power than the physiological monitor 2006 that acquires the data.

The user device 2020 may include any computing device as described herein, including without limitation a smartphone, a desktop computer, a laptop computer, a network computer, a tablet, a mobile device, a portable digital assistant, a cellular phone, a portable media or entertainment device, and so on. The user device 2020 may provide a user interface 2022 for access to data and analysis by a user, and/or to control operation of the physiological monitor 2006. The user interface 2022 may be maintained by a locally-executing application on the user device 2020, or the user interface 2022 may be remotely served and presented on the user device 2020, e.g., from the remote server 2030 or the one or more other resources 2050.

In general, the remote server 2030 may include data storage, a network interface, and/or other processing circuitry. The remote server 2030 may process data from the physiological monitor 2006 and perform infection monitoring/analyses or any of the other analyses described herein, and may host a user interface for remote access to this data, e.g., from the user device 2020. The remote server 2030 may include a web server or other programmatic front end that facilitates web-based access by the user devices 2020 or the physiological monitor 2006 to the capabilities of the remote server 2030 or other components of the system 2000.

The other resources 2050 may include any resources that can be usefully employed in the devices, systems, and methods as described herein. For example, these other resources 2050 may include without limitation other data networks, human actors (e.g., programmers, researchers, annotators, editors, analysts, and so forth), sensors (e.g., audio or visual sensors), data mining tools, computational tools, data monitoring tools, algorithms, and so forth. The other resources 2050 may also or instead include any other software or hardware resources that may be usefully employed in the networked applications as contemplated herein. For example, the other resources 2050 may include payment processing servers or platforms used to authorize payment for access, content, or option/feature purchases, or otherwise. In another aspect, the other resources 2050 may include certificate servers or other security resources for third-party verification of identity, encryption or decryption of data, and so forth. In another aspect, the other resources 2050 may include a desktop computer or the like co-located (e.g., on the same local area network with, or directly coupled to through a serial or USB cable) with a user device 2020, wearable strap 2010, or remote server 2030. In this case, the other resources 2050 may provide supplemental functions for components of the system 2000.

The other resources 2050 may also or instead include one or more web servers that provide web-based access to and from any of the other participants in the system 2000. While depicted as a separate network entity, it will be readily appreciated that the other resources 2050 (e.g., a web server) may also or instead be logically and/or physically associated with one of the other devices described herein, and may for example, include or provide a user interface 2022 for web access to a remote server 2030 or a database in a manner that permits user interaction through the data network 2002, e.g., from the physiological monitor 2006 or the user device 2020.

In one aspect, the system 2000 may include a server 2030 configured to receive heart rate data from a wearable physiological monitor 2006 and to evaluate the health (e.g., a respiratory health) of a wearer of the physiological monitor 2006 by performing the steps of: determining a historical respiratory rate pattern for the user at a first number of predetermined daily intervals based on the heart rate data, the historical respiratory rate pattern characterizing one or more features of a typical respiratory rate pattern during the one or more predetermined daily intervals; determining a current respiratory rate pattern for the user during a second predetermined daily interval based on the heart rate data; evaluating the one or more features of the current respiratory rate pattern; comparing the one or more features of the current respiratory rate pattern to the one or more features of the typical respiratory rate pattern; and in response to a predetermined difference between the one or more features of the current respiratory rate pattern and the one or more features of the typical respiratory rate pattern, creating an indicator of a likelihood of a respiratory infection of the user.

The system 2000 may include a wearable physiological monitor configured to continuously acquire heart rate data from the user and transmit the heart rate data to the server. The system 2000 may also or instead include a user device configured to receive an alert from the server and display the alert to the user when the likelihood of the respiratory infection is above a predetermined threshold.

It will be understood that one or more of the steps related to techniques for infection monitoring as described herein, or sub-steps, calculations, functions, and the like related thereto, can be performed locally, remotely, or some combination of these. Thus, the one or more of the steps of the methods 1800, 1900 of Figs. 18-19 may be performed locally on a wearable device, remotely on a server or other remote resource, on an intermediate device such as a local computer used by the user to access the remote resource, or any combination of these. For example, using the example system 2000 of Fig. 20, one or more steps of a technique for infection monitoring may, wholly or partially, be performed locally on one or more of the physiological monitor 2006 and the user device 2020, such as by training a machine learning model to detect deviations from a typical respiratory rate pattern, and then pruning or otherwise optimizing the machine learning model for deployment on the wearable device. Also, or instead, one or more steps of a technique for infection monitoring may, wholly or partially, be performed remotely on one or more of the remote server 2030 and the other resource(s) 2050. Thus, for example, where a wearable monitor is positioned sufficiently near a smartphone of the user for short range wireless communications during sleep, heart rate data may be continuously or periodically transmitted to the remote server 2030, which may monitor received data to monitor for infections by detecting deviations from a typical respiratory rate pattern. Other combinations are also possible.

Example Study

Additional details of a technique for generating a daily indicator of health status, including a quantitative daily indicator of a likelihood of a Covid-19 infection, are described by way of non-limiting examples in Miller, et al., "Analyzing changes in respiratory rate to predict the risk of COVID-19 infection," PLoS ONE 15(12): e0243693 (December 10, 2020), available at https://doi.org/10.1371/journal.pone.0243693 and hereinafter referred to as the "Miller Paper". This study shows that infection monitoring using, at least in part, a wearable physiological monitoring device such as any described herein, enables improved early detection of diseases such as COVID-19, the disease caused by the SARS-CoV-2 virus, which can cause shortness of breath, lung damage, and impaired respiratory function. Containing this virus has proven difficult, in large part due to its high transmissibility during the pre-symptomatic incubation. However, changes in respiratory rate could serve as a leading indicator of SARS-CoV-2 infections as demonstrated by the example study in the Miller Paper.

The novel coronavirus disease (COVID-19) is caused by the severe acute respiratory syndrome coronavirus 2 (SARS-CoV-2) virus and predominantly presents as a lower respiratory tract infection. Severe cases of the disease can result in alveolar damage and progressive respiratory failure. Respiratory rate is a common screening tool to identify lower respiratory tract infections in clinical settings, where guidelines define tachypnea as a respiratory rate greater than 20 respirations per minute (rpm), and generally advise further tests (e.g., chest radiography) when present (see, e.g., Ebell, M.H., "Predicting pneumonia in adults with respiratory illness," Am. Fam. Physician, 76(4):560-2, pmid: 17853631 (2007)). While such thresholds are useful in clinical settings, they are usually only implemented once symptoms have emerged and are not sensitive to individual variations in normal respiratory function. Given that COVID-19 impairs and damages the respiratory system, changes in respiratory efficiency-and therefore resting respiratory rate-may occur in the early stages of infection. In this context, noninvasive daily monitoring of respiratory rate may be used to detect subclinical intraindividual deviations and identify potential infections that would otherwise be overlooked by clinical thresholds.

Using the techniques described herein of the present teachings, the example study in the Miller Paper was conducted to determine the effectiveness of detecting COVID-19 infection using data from wearable physiological monitoring devices. In this study, a total of 271 individuals (age = 37.3 ± 9.5, 190 male, 81 female) who experienced symptoms consistent with COVID-19 were included, where 81 tested positive for SARS-CoV-2 and 190 tested negative; these 271 individuals collectively contributed 2672 samples (days) of data (1856 healthy days, 231 while infected with COVID-19 and 585 while negative for COVID-19 but experiencing symptoms). To train an algorithm, individuals were segmented as follows; (1) a training dataset of individuals who tested positive for COVID-19 (n = 57 people, 537 samples); (2) a validation dataset of individuals who tested positive for COVID-19 (n = 24 people, 320 samples); (3) a validation dataset of individuals who tested negative for COVID-19 (n = 190 people, 1815 samples). All data was extracted from the wearable physiological monitoring devices and systems such as those described herein-and in particular, from data from a wrist-worn strap to produce validated estimates of respiratory rate and other physiological measures. Using the training dataset, a model as described herein was developed to estimate the probability of SARS-CoV-2 infection based on changes in respiratory rate during night-time sleep. The model's ability to identify COVID-positive individuals not used in training and robustness against COVID-negative individuals with similar symptoms were examined for a six-day period spanning the onset of symptoms. The model identified 20% of COVID-19 positive individuals in the validation dataset in the two days prior to symptom onset, and 80% of COVID-19 positive cases by the third day of symptoms.

A respiratory rate, resting heart rate (RHR) and heart rate variability (HRV) were measured using a wearable physiological monitor in the form of a wrist-worn device, such as those described herein. In particular, the wearable physiological monitor used in the study included a small, waterproof, and rechargeable device containing a photoplethysmogram, accelerometer, thermometer, capacitive touch sensor, and gyroscope that can be worn relatively comfortably 24-hours per day and that can last at least 5 days between charges.

The following physiological data was obtained: respiratory rate in the form of a median value of respirations per minute, derived each night during the main sleep period via photoplethysmography; RHR in the form of average beats per minute sampled during the last five minutes of the last episode of slow wave sleep each night; and HRV sampled during the last five minutes of the last episode of slow wave sleep each night using the root mean square of successive RR interval differences (rMSSD) method in units of milliseconds. In addition to automated tracking of physiological data, the application used supported tracking of manually reported contextual factors such as COVID-19 symptoms and test results.

Respiratory rate was evaluated as a potentially sensitive indicator of infection due to observations of low internight variation. To support the use of this metric in the model, a supplementary dataset from November 2019 was generated for analysis. A date range of 1 November 2019 through 30 November 2019 was chosen to avoid confounding factors related to the COVID-19 pandemic. A total of 25,000 individuals were randomly selected (n = 750,000 nights), where the only inclusion criteria was having respiratory rate recorded on all 30 consecutive nights. Resting heart rate and resting heart rate variability over this period were included for comparison. The following variables were calculated from the November dataset for each of the physiological metrics: mean intraindividual mean, i.e., mean within-member means; standard deviation of intraindividual means, i.e., standard deviation of within-member means; mean intraindividual standard deviation, i.e., mean within-member standard deviation; standard deviation of intraindividual standard deviations, i.e., standard deviation of within-member standard deviations; and coefficient of variation, i.e., intraindividual standard deviation divided by the intraindividual mean.

A total of 271 adults (age = 37.3 ± 9.5, 190 male, 81 female) were included in the example study. Inclusion criteria were (1) self-reporting symptoms consistent with COVID-19 (e.g., cough, fever, and/or fatigue) and (2) having been tested for the SARS-CoV-2 virus. These individuals were separated into three groups: a training dataset of COVID-19 positive individuals who began experiencing COVID-19 symptoms between 14 March 2020 and 14 April 2020 (n = 57); a first validation dataset of COVID-19 positive individuals who began experiencing COVID-19 symptoms between 14 April and 6 June 2020 (n = 24); and a second validation dataset of individuals who experienced COVID-19 symptoms but reported a negative test result (n = 190). In order to develop the algorithm, data was categorized by day relative to symptom onset (day 0) into: healthy days (data extracted from 30 to 14 days prior to symptom onset), and infected days (data extracted between 2 days prior to symptom onset and 3 days post symptom onset).

All 271 individuals contributed to both categories, with a maximum of 15 healthy days per person and 6 infected days per person. For the training dataset, 146 infected days and 391 healthy days were included. Due to the class imbalance between infected days and healthy days, synthetic samples (i.e., days) were generated for the positive class (i.e., infected days) by adding uniformly distributed random noise on the interval [0, 1) to each infected day, bringing the number of infected days to 292. Generation of synthetic samples was done only for training and was not repeated for the validation datasets. Synthetic samples were only used for training the model and were excluded from the analysis of the training set presented throughout. For the first validation dataset, 85 infected days and 235 healthy days were included. For the second validation dataset, 585 infected days and 1230 healthy days were included.

The daily respiratory rate value (hereinafter current value) for each individual was transformed into features based on how it compared to the values taken on each of the 21 days prior. In all datasets, only current values for which the prior 21 consecutive nights' respiratory rates were available were included. These features capture the dynamics of deviation from recent trends along a variety of time scales. In generating the classifier's features, the following metrics were used: $RR_0$, which is a current value (a respiratory rate); $\tilde{x}$, which is a median of the respiratory rates in the 14 day period between 21 and 7 nights prior to the current value; $\sigma$, which is a standard deviation of the respiratory rates in the 14 day period between 21 and 7 nights prior to the current value; $\mu_2$, which is a mean of the current value and immediately prior night's respiratory rate; $\mu_3$, which is a mean of the current value and immediately prior two nights' respiratory rates; $\mu_6$, which is a median of the immediately prior 6 nights of respiratory rates, excluding $RR_0$; and $m_6$, which is a slope of the linear regression of the collection of the respiratory rates of the current day to 6 days prior, excluding $RR_0$. The features derived from these metrics were as follows:

1.

$$\mu_2/\tilde{x}$$

2.

$$(\mu_2 - \tilde{x})/\sigma$$

3.

$$RR_0 - \mu_3$$

4. $m_6$

5.

$$\mu_2 - \mu_6$$

Collectively, these features capture dynamics of the changes in respiratory rate over time. A modified z-score (i.e., utilizing a median value rather than mean) was used to create a baseline that is robust to outlier values and more stable over the short time periods explored in the example study. Using a lagged baseline, as in $\tilde{x}$ in features 1 and 2, allows data to increase during an incubation period without artificially elevating the baseline and masking the impact of the SARS-CoV-2 infection. A gradient boosted classifier (see, e.g., Pedregosa F., et al., "Scikit-learn: Machine Learning in Python," J. Mach. Learn. Res., 12:2825-30 (2011)) was trained using Python Language Software (version 3.6.2) on the derived features to return a probability of SARS-CoV-2 infection on healthy and infected days.

In order to evaluate the model's performance for classifying healthy and infected days, a threshold value was assigned to the probability output of the model such that meeting or exceeding that threshold was equivalent to classifying healthy or infected days as COVID-19 positive (C+); while failing to exceed the threshold was equivalent to classifying healthy or infected days as COVID-19 negative (C-). The threshold value was strategically set at 0.3 to maximize the utility of the model by reducing the chance of false negatives at the expense of increasing false positives, in recognition that false negatives may have higher costs to society than false positives. The model's performance for classifying healthy days and infected days for each dataset was also evaluated at that threshold by calculating sensitivity, specificity, positive predictive value (PPV), and negative predictive value (NPV).

The aim of the example study was to assess the ability of an algorithm according to an aspect of the present teachings to classify changes in respiratory rate, as indicative of COVID-19 infection immediately prior to and during the first days of symptoms. Some findings of the study were: (1) the stability of nightly respiratory rate measurements within healthy individuals can make it a useful metric for tracking changes in wellness; (2) the model may be capable of distinguishing between healthy days and infected days for individuals who tested positive to COVID-19 as well as those who had symptoms but tested negative; and (3) the model identified 20% of individuals of COVID-19 positive prior to the onset of symptoms, and correctly identified 80% of COVID-19 positive individuals by the third day of symptoms. These findings show that while interindividual variation in nightly respiratory rate can be large, intraindividual variability across 30 nights is typically quite small, with mean intraindividual standard deviation of 0.51 ± 0.20 rpm. The finding that nighttime median respiratory rate in healthy individuals has low internight variability suggests that deviations in respiratory rate may be a useful indicator of acute changes in lower respiratory tract health.

Thus, the study shows that a predictive algorithm according to an aspect of the present teachings can leverage individual baseline data and use nightly respiratory rate (when contextualized by 21-day trends) to predict COVID-19 infections. The study indicates that the final stages of incubation and early stages of the infection have a detectable signature that can identify individuals who should self-isolate and seek testing. This approach is particularly advantageous for individuals with low resting respiratory rates, who despite experiencing significantly elevated respiratory rates relative to their personal baseline, might not be medically classified as tachypneic according to globally defined norms.

There are many practical applications for the model's ability to analyze daily changes in respiratory rate (or other physiological signals), including aiding testing protocols and monitoring essential workers. Repeated screening for an individual may be costly and impractical. Given the performance of the model at discriminating between COVID-19 and other illnesses with similar symptomatology, the model can be used to streamline or triage testing protocols in areas that may have testing kit shortages. In addition, the algorithm may be particularly advantageous in situations where physical distancing is impractical (e.g., industry, elite sport, healthcare, and so on), but where a positive COVID-19 case could have major implications. Thus, along with recommended hygiene and physical distancing protocols, wearable technologies according to the present teachings can be used as a point of care measure to monitor employees and/or athletes during the transition back to work and competition.

It should be noted that the sensitivity and specificity of the model may be determined both by the discriminatory power of the features and by the threshold selected to discriminate between COVID-19 positive and COVID-19 negative designations. For example, healthy days may tend to be assigned lower probabilities of being COVID-19 positive while infected days may tend to be assigned higher probabilities. For the same probability distributions, a higher threshold may result in higher specificity but lower sensitivity, while a lower threshold may make the opposite tradeoff increasing sensitivity while decreasing specificity. The optimal threshold for a given model may be dependent on its intended application—e.g., while a threshold of 0.5 would maximize accuracy, this is often not the metric most associated with practical utility. For the algorithm presented in the example study, a false positive-indicating that COVID-19 negative individual may be COVID-19 positive-can mean that an individual self-isolates unnecessarily, while a false negative-indicating that a COVID-19 positive individual is COVID-19 negative-could result in the individual interacting with and potentially infecting others. Therefore, the reduced threshold value of 0.3 was chosen in the study in recognition that false negatives have higher costs to society than false positives. However, other thresholds may also or instead be applied according to the intended audience and use of the test.

Overall, the study described above validates infection testing based on continuous physiological monitoring of heart rate data as a non-invasive method for detecting SARS-CoV-2 infection prior to and during the first days of symptoms. The early stages of the infection have a detectable signature that can help to identify individuals who should self-isolate and/or seek testing.

The above systems, devices, methods, processes, and the like may be realized in hardware, software, or any combination of these suitable for the control, data acquisition, and data processing described herein. This includes realization in one or more microprocessors, microcontrollers, embedded microcontrollers, programmable digital signal processors or other programmable devices or processing circuitry, along with internal and/or external memory. This may also, or instead, include one or more application specific integrated circuits, programmable gate arrays, programmable array logic components, or any other device or devices that may be configured to process electronic signals. It will further be appreciated that a realization of the processes or devices described above may include computer-executable code created using a structured programming language such as C, an object oriented programming language such as C++, or any other high-level or low-level programming language (including assembly languages, hardware description languages, and database programming languages and technologies) that may be stored, compiled or interpreted to run on one of the above devices, as well as heterogeneous combinations of processors, processor architectures, or combinations of different hardware and software.

Thus, in one aspect, each method described above, and combinations thereof may be embodied in computer executable code that, when executing on one or more computing devices, performs the steps thereof. In another aspect, the methods may be embodied in systems that perform the steps thereof, and may be distributed across devices in a number of ways, or all of the functionality may be integrated into a dedicated, standalone device or other hardware. The code may be stored in a non-transitory fashion in a computer memory, which may be a memory from which the program executes (such as random access memory associated with a processor), or a storage device such as a disk drive, flash memory or any other optical, electromagnetic, magnetic, infrared or other device or combination of devices. In another aspect, any of the systems and methods described above may be embodied in any suitable transmission or propagation medium carrying computer-executable code and/or any inputs or outputs from same. In another aspect, means for performing the steps associated with the processes described above may include any of the hardware and/or software described above.

## Claims

1.  A computer program product comprising computer executable code embodied in a non-transitory computer readable medium that, when executing on one or more computing devices, performs the steps of:

    acquiring heart rate data from a user with a wearable physiological monitor 2006;
    determining a historical respiratory rate pattern for the user at a first number of predetermined daily intervals during periods of deep sleep by the user based on the heart rate data, the historical respiratory rate pattern characterizing one or more features of a respiratory activity of the user during the first number of predetermined daily intervals during periods of deep sleep by the user;
    determining a current respiratory rate pattern for the user during a second predetermined daily interval based on the heart rate data during a second period of deep sleep;
    evaluating the one or more features of the current respiratory rate pattern;
    comparing the one or more features of the current respiratory rate pattern to the one or more features of the historical respiratory rate pattern; and
    in response to a predetermined difference between the one or more features of the current respiratory rate pattern and the one or more features of the historical respiratory rate pattern, creating an indicator of a likelihood of a respiratory infection of the user.

**2.** The computer program product of claim 1, wherein the first number of predetermined daily intervals and the second predetermined daily interval include sleep intervals detected using data using the wearable physiological monitor 2006.

**3.** The computer program product of claim 1, wherein comparing the one or more features is performed on a remote server 2030.

**4.** The computer program product of claim 3, wherein the indicator is transmitted from the remote server 2030 to a device associated with the user.

**5.** The computer program product of claim 1, wherein the historical respiratory rate pattern includes a respiratory rate at a certain daily time interval and wherein the current respiratory rate pattern includes a current respiratory rate at a corresponding daily time.

**6.** The computer program product of claim 2, wherein the sleep intervals include periods of deep sleep most immediately preceding an end of a sleep event.

**7.** The computer program product of claim 1, further comprising code that performs the steps of:

training a machine classifier to return a probability that a set of values for the one or more features is indicative of the respiratory infection; and
applying the machine classifier to the one or more features of the current respiratory rate pattern during the second predetermined daily interval.

**8.** The computer program product of claim 1, wherein the wearable physiological monitor 2006 acquires the heart rate data using photoplethysmography, and/or wherein the historical respiratory rate pattern and the current respiratory rate pattern are determined at least in part by correlating variations in heart rate to inhalation and exhalation.

**9.** The computer program product of claim 1, further comprising code that performs the steps of:

creating the indicator at a server; and
transmitting the indicator to a device associated with the user for display.

**10.** The computer program product of claim 1, further comprising code that performs the steps of:

creating the indicator on the wearable physiological monitor 2006; and
transmitting the indicator to a device associated with the user.

**11.** The computer program product of claim 1, wherein the first number of predetermined daily intervals includes a number of intervals sufficient to establish a pre-infection baseline for a health respiratory pattern and/or wherein the first number of predetermined daily intervals and the second predetermined daily interval include intervals selected based on at least quality and consistency of the heart rate data.

**12.** The computer program product of claim 1, further comprising detecting the periods of deep sleep based on the heart rate data from the user.

**13.** The computer program product of claim 1, further comprising detecting the periods of deep sleep by detecting slow wave sleep using data from the wearable physiological monitor including at least one of body temperature data, galvanic skin response data, acoustic data, and motion data.

**14.** A system, comprising:
a server 2030 including one or more processors configured to receive heart rate data from a user with a wearable physiological monitor and to evaluate a respiratory health of a user by performing the steps of:

determining a historical respiratory rate pattern for the user at a first number of predetermined daily intervals based on the heart rate data during deep sleep, the historical respiratory rate pattern characterizing one or more features of a typical respiratory rate pattern during the one or more predetermined daily intervals during deep sleep;

determining a current respiratory rate pattern for the user during a second predetermined daily interval based on the heart rate data during a period of deep sleep;

evaluating the one or more features of the current respiratory rate pattern; comparing the one or more features of the current respiratory rate pattern to the one or more features of the typical respiratory rate pattern; and

in response to a predetermined difference between the one or more features of the current respiratory rate pattern and the one or more features of the typical respiratory rate pattern, creating an indicator of a likelihood of a respiratory infection of the user.

**15.** The system of claim 14, further comprising a wearable physiological monitor 2006 configured to continuously acquire heart rate data from the user and transmit the heart rate data to the server.

**16.** The system of claim 14, further comprising a user device configured to receive an alert from the server and display the alert to the user when the likelihood of the respiratory infection is above a predetermined threshold.

**Patentansprüche**

**1.** Computerprogrammprodukt, das einen ausführbaren Computercode umfasst, der in einem nicht flüchtigen computerlesbaren Medium verkörpert ist, das bei der Ausführung auf einem oder mehreren Computergeräten die folgenden Schritte ausführt:

Erfassen von Herzfrequenzdaten von einem Benutzer mit einem tragbaren physiologischen Monitor 2006;

Bestimmung eines historischen Atemfrequenzmusters für den Benutzer in einer ersten Anzahl von vorbestimmten täglichen Intervallen während Tiefschlafperioden des Benutzers auf der Grundlage der Herzfrequenzdaten, wobei das historische Atemfrequenzmuster ein oder mehrere Merkmale einer Atemaktivität des Benutzers während der ersten Anzahl von vorbestimmten täglichen Intervallen während Tiefschlafperioden des Benutzers kennzeichnet;

Bestimmung eines aktuellen Atemfrequenzmusters für den Benutzer während eines zweiten vorbestimmten täglichen Intervalls auf der Grundlage der Herzfrequenzdaten während einer zweiten Tiefschlafperiode;

Auswerten eines oder mehrerer Merkmale des aktuellen Atemfrequenzm usters;

Vergleichen des einen oder der mehreren Merkmale des aktuellen Atemfrequenzmusters mit dem einen oder den mehreren Merkmalen des historischen Atemfrequenzmusters; und

Erstellen eines Indikators für die Wahrscheinlichkeit einer Atemwegsinfektion des Benutzers als Reaktion auf eine vorbestimmte Differenz zwischen dem einen oder den mehreren Merkmalen des aktuellen Atemfrequenzmusters und dem einen oder den mehreren Merkmalen des historischen Atemfrequenzmusters.

**2.** Computerprogrammprodukt nach Anspruch 1, wobei die erste Anzahl vorbestimmter täglicher Intervalle und das zweite vorbestimmte tägliche Intervall Schlafintervalle umfassen, die unter Verwendung von Daten unter Verwendung des tragbaren physiologischen Monitors 2006 erfasst werden.

**3.** Computerprogrammprodukt nach Anspruch 1, wobei das Vergleichen des einen oder der mehreren Merkmale auf einem entfernten Server 2030 durchgeführt wird.

**4.** Computerprogrammprodukt nach Anspruch 3, wobei der Indikator von dem entfernten Server 2030 an ein mit dem Benutzer verbundenes Gerät übertragen wird.

**5.** Computerprogrammprodukt nach Anspruch 1, wobei das historische Atemfrequenzmuster eine Atemfrequenz in einem bestimmten täglichen Zeitintervall umfasst und wobei das aktuelle Atemfrequenzmuster eine aktuelle Atemfrequenz zu einer entsprechenden täglichen Zeit umfasst.

**6.** Computerprogrammprodukt nach Anspruch 2, wobei die Schlafintervalle Tiefschlafperioden umfassen, die einem Ende eines Schlafereignisses am unmittelbarsten vorausgehen.

**7.** Computerprogrammprodukt nach Anspruch 1, das ferner einen Code umfasst, der die folgenden Schritte ausführt:

Trainieren eines maschinellen Klassifikators, um eine Wahrscheinlichkeit zurückzugeben, dass ein Satz von Werten für das eine oder die mehreren Merkmale auf die Atemwegsinfektion hindeutet; und

Anwendung des maschinellen Klassifikators auf das eine oder die mehreren Merkmale des aktuellen Atemfrequenzmusters während des zweiten vorbestimmten täglichen Intervalls.

8. Computerprogrammprodukt nach Anspruch 1, wobei der tragbare physiologische Monitor 2006 die Herzfrequenzdaten mittels Photoplethysmographie erfasst und/oder wobei das historische Atemfrequenzmuster und das aktuelle Atemfrequenzmuster zumindest teilweise durch Korrelieren von Herzfrequenzschwankungen mit dem Einatmen und Ausatmen bestimmt werden.

9. Computerprogrammprodukt nach Anspruch 1, das ferner einen Code umfasst, der die folgenden Schritte ausführt:

   Erstellen des Indikators auf einem Server; und
   Übertragen des Indikators an ein mit dem Benutzer verbundenes Gerät zur Anzeige.

10. Computerprogrammprodukt nach Anspruch 1, das ferner einen Code umfasst, der die folgenden Schritte ausführt:

   Erstellen des Indikators auf dem tragbaren physiologischen Monitor 2006; und
   Übertragen des Indikators an ein mit dem Benutzer verbundenes Gerät.

11. Computerprogrammprodukt nach Anspruch 1, wobei die erste Anzahl vorbestimmter täglicher Intervalle eine Anzahl von Intervallen umfasst, die ausreicht, um eine Basislinie vor einer Infektion für ein gesundes Atemmuster festzulegen, und/oder wobei die erste Anzahl vorbestimmter täglicher Intervalle und das zweite vorbestimmte tägliche Intervall Intervalle umfassen, die auf der Grundlage von zumindest der Qualität und Konsistenz der Herzfrequenzdaten ausgewählt werden.

12. Computerprogrammprodukt nach Anspruch 1, das ferner das Erfassen der Tiefschlafperioden auf der Grundlage der Herzfrequenzdaten des Benutzers umfasst.

13. Computerprogrammprodukt nach Anspruch 1, das ferner das Erfassen der Tiefschlafperioden durch Erfassen des langsamen Wellenschlafs unter Verwendung von Daten von dem tragbaren physiologischen Monitor umfasst, der mindestens eines der folgenden Daten umfasst: Körpertemperaturdaten, Daten der galvanischen Hautreaktion, akustische Daten und Bewegungsdaten.

14. System, das Folgendes umfasst:
   einen Server 2030 mit einem oder mehreren Prozessoren, die so konfiguriert sind, dass sie Herzfrequenzdaten von einem Benutzer mit einem tragbaren physiologischen Monitor empfangen und die Atmungsgesundheit eines Benutzers durch Ausführen der folgenden Schritte bewerten:

   Bestimmen eines historischen Atemfrequenzmusters für den Benutzer bei einer ersten Anzahl von vorbestimmten täglichen Intervallen auf der Grundlage der Herzfrequenzdaten während des Tiefschlafs, wobei das historische Atemfrequenzmuster ein oder mehrere Merkmale eines typischen Atemfrequenzmusters während des einen oder der mehreren vorbestimmten täglichen Intervalle während des Tiefschlafs charakterisiert;
   Bestimmung eines aktuellen Atemfrequenzmusters für den Benutzer während eines zweiten vorbestimmten täglichen Intervalls basierend auf den Herzfrequenzdaten während einer Tiefschlafperiode;
   Auswertung des einen oder der mehreren Merkmale des aktuellen Atemfrequenzmusters; Vergleich des einen oder der mehreren Merkmale des aktuellen Atemfrequenzmusters mit dem einen oder den mehreren Merkmalen des typischen Atemfrequenzmusters; und
   als Reaktion auf eine vorbestimmte Differenz zwischen dem einen oder den mehreren Merkmalen des aktuellen Atemfrequenzmusters und dem einen oder den mehreren Merkmalen des typischen Atemfrequenzmusters einen Indikator für die Wahrscheinlichkeit einer Atemwegsinfektion des Benutzers zu erstellen.

15. System nach Anspruch 14, das ferner einen tragbaren physiologischen Monitor 2006 umfasst, der so konfiguriert ist, dass er kontinuierlich Herzfrequenzdaten von dem Benutzer erfasst und die Herzfrequenzdaten an den Server überträgt.

16. System nach Anspruch 14, das ferner ein Benutzergerät umfasst, das so konfiguriert ist, dass es eine Warnung vom Server empfängt und dem Benutzer anzeigt, wenn die Wahrscheinlichkeit einer Atemwegsinfektion über einem vorgegebenen Schwellenwert liegt.

**Revendications**

1. Produit-programme informatique comprenant un code exécutable par ordinateur incorporé dans un support lisible par ordinateur non transitoire qui, lors de l'exécution sur un ou plusieurs dispositifs informatiques, réalise les étapes consistant à :

   acquérir des données de fréquence cardiaque provenant d'un utilisateur avec un moniteur physiologique portable (2006) ;
   déterminer un modèle de fréquence respiratoire historique pour l'utilisateur à un premier nombre d'intervalles quotidiens prédéterminés pendant des périodes de sommeil profond par l'utilisateur sur la base des données de fréquence cardiaque, le modèle de fréquence respiratoire historique caractérisant une ou plusieurs caractéristiques d'une activité respiratoire de l'utilisateur pendant le premier nombre d'intervalles quotidiens prédéterminés pendant des périodes de sommeil profond par l'utilisateur ;
   déterminer un modèle de fréquence respiratoire en cours pour l'utilisateur pendant un deuxième intervalle quotidien prédéterminé sur la base des données de fréquence cardiaque pendant une deuxième période de sommeil profond ;
   évaluer l'une ou plusieurs caractéristiques du modèle de fréquence respiratoire en cours ;
   comparer l'une ou plusieurs caractéristiques du modèle de fréquence respiratoire en cours à l'une ou plusieurs caractéristiques du modèle de fréquence respiratoire historique ; et
   en réponse à une différence prédéterminée entre l'une ou plusieurs caractéristiques du modèle de fréquence respiratoire en cours et l'une ou plusieurs caractéristiques du modèle de fréquence respiratoire historique, créer un indicateur d'une probabilité d'une infection respiratoire de l'utilisateur.

2. Produit-programme informatique selon la revendication 1, dans lequel le premier nombre d'intervalles quotidiens prédéterminés et le deuxième intervalle quotidien prédéterminé comportent des intervalles de sommeil détectés à l'aide de données à l'aide du moniteur physiologique portable (2006).

3. Produit-programme informatique selon la revendication 1, dans lequel la comparaison d'une ou plusieurs caractéristiques est réalisée sur un serveur distant (2030).

4. Produit-programme informatique selon la revendication 3, dans lequel l'indicateur est transmis du serveur distant (2030) à un dispositif associé à l'utilisateur.

5. Produit-programme informatique selon la revendication 1, dans lequel le modèle de fréquence respiratoire historique comporte une fréquence respiratoire à un certain intervalle de temps quotidien et dans lequel le modèle de fréquence respiratoire en cours comporte une fréquence respiratoire en cours à un moment quotidien correspondant.

6. Produit-programme informatique selon la revendication 2, dans lequel les intervalles de sommeil comportent des périodes de sommeil profond précédant le plus immédiatement une fin d'un événement de sommeil.

7. Produit-programme informatique selon la revendication 1, comprenant en outre un code qui réalise les étapes consistant à :

   entraîner un classificateur automatique pour renvoyer une probabilité qu'un ensemble de valeurs pour l'une ou plusieurs caractéristiques indique l'infection respiratoire ; et
   appliquer le classificateur automatique à l'une ou plusieurs caractéristiques du modèle de fréquence respiratoire en cours pendant le deuxième intervalle quotidien prédéterminé.

8. Produit-programme informatique selon la revendication 1, dans lequel le moniteur physiologique portable (2006) acquiert les données de fréquence cardiaque à l'aide de la photopléthysmographie, et/ou dans lequel le modèle de fréquence respiratoire historique et le modèle de fréquence respiratoire en cours sont déterminés au moins en partie par la corrélation de variations de la fréquence cardiaque à l'inspiration et à l'expiration.

9. Produit-programme informatique selon la revendication 1, comprenant en outre un code qui réalise les étapes consistant à :

   créer l'indicateur au niveau d'un serveur ; et
   transmettre l'indicateur à un dispositif associé à l'utilisateur pour affichage.

**10.** Produit-programme informatique selon la revendication 1, comprenant en outre un code qui réalise les étapes consistant à :
créer l'indicateur sur le moniteur physiologique portable (2006) ; et transmettre l'indicateur à un dispositif associé à l'utilisateur.

**11.** Produit-programme informatique selon la revendication 1, dans lequel le premier nombre d'intervalles quotidiens prédéterminés comporte un nombre d'intervalles suffisant pour établir une ligne de base de pré-infection pour un modèle respiratoire de santé et/ou dans lequel le premier nombre d'intervalles quotidiens prédéterminés et le deuxième intervalle quotidien prédéterminé comportent des intervalles sélectionnés sur la base au moins de la qualité et de la cohérence des données de fréquence cardiaque.

**12.** Produit-programme informatique selon la revendication 1, consistant en outre à détecter les périodes de sommeil profond sur la base des données de fréquence cardiaque provenant de l'utilisateur.

**13.** Produit-programme informatique selon la revendication 1, consistant en outre à détecter les périodes de sommeil profond par la détection d'un sommeil à ondes lentes à l'aide de données provenant du moniteur physiologique portable comportant au moins l'un parmi les données de température corporelle, des données de réponse galvanique cutanée, des données acoustiques et des données de mouvement.

**14.** Système, comprenant :
un serveur (2030) comportant un ou plusieurs processeurs configurés pour recevoir des données de fréquence cardiaque provenant d'un utilisateur avec un moniteur physiologique portable et pour évaluer la santé respiratoire d'un utilisateur par la réalisation des étapes consistant à :

déterminer un modèle de fréquence respiratoire historique pour l'utilisateur à un premier nombre d'intervalles quotidiens prédéterminés sur la base des données de fréquence cardiaque pendant le sommeil profond, le modèle de fréquence respiratoire historique caractérisant une ou plusieurs caractéristiques d'un modèle de fréquence respiratoire typique pendant l'un ou plusieurs intervalles quotidiens prédéterminés pendant le sommeil profond ;
déterminer un modèle de fréquence respiratoire en cours pour l'utilisateur pendant un deuxième intervalle quotidien prédéterminé sur la base des données de fréquence cardiaque pendant une période de sommeil profond ;
évaluer l'une ou plusieurs caractéristiques du modèle de fréquence respiratoire en cours ; comparer l'une ou plusieurs caractéristiques du modèle de fréquence respiratoire en cours à l'une ou plusieurs caractéristiques du modèle de fréquence respiratoire typique ; et
en réponse à une différence prédéterminée entre l'une ou plusieurs caractéristiques du modèle de fréquence respiratoire en cours et l'une ou plusieurs caractéristiques du modèle de fréquence respiratoire typique, créer un indicateur d'une probabilité d'une infection respiratoire de l'utilisateur.

**15.** Système selon la revendication 14, comprenant en outre un moniteur physiologique portable (2006) configuré pour acquérir en continu des données de fréquence cardiaque provenant de l'utilisateur et transmettre les données de fréquence cardiaque au serveur.

**16.** Système selon la revendication 14, comprenant en outre un dispositif utilisateur configuré pour recevoir une alerte en provenance du serveur et afficher l'alerte à l'utilisateur lorsque la probabilité de l'infection respiratoire est supérieure à un seuil prédéterminé.

*Fig. 1*

EP 4 165 658 B1

*Fig. 3*

*Fig. 2*

*Fig. 4*

*Fig. 5*

```
                                                                    ⌐602
┌─────────────────────────────────────────────────────────────┐
│           Emit light using light emitters toward user's skin │
└─────────────────────────────────────────────────────────────┘
                              │
                              ▼                                     ⌐604
┌─────────────────────────────────────────────────────────────┐
│        Detect light reflected from user's skin using light detectors │
└─────────────────────────────────────────────────────────────┘
                              │
                              ▼                                     ⌐606
┌─────────────────────────────────────────────────────────────┐
│           Pre-process signals associated with reflected light │
└─────────────────────────────────────────────────────────────┘
                              │
                              ▼                                     ⌐608
┌─────────────────────────────────────────────────────────────┐
│   Execute peak detection algorithm to detect peaks in pre-processed signals │
└─────────────────────────────────────────────────────────────┘
                              │
                              ▼                                     ⌐610
┌─────────────────────────────────────────────────────────────┐
│           Determine an RR interval based on detected peaks │
└─────────────────────────────────────────────────────────────┘
                              │
                              ▼                                     ⌐612
┌─────────────────────────────────────────────────────────────┐
│       Determine confidence level associated with RR interval │
└─────────────────────────────────────────────────────────────┘
                              │
                              ▼                                     ⌐614
                  Yes  ◁──────  Confidence level  ──────▷  No
                              > threshold?
```

Use detected peaks to determine instantaneous heart rate ⌐616

Determine heart rate variability ⌐618

Execute frequency analysis algorithm to determine instantaneous heart rate based on pre-processed signals associated with reflected light ⌐620

Determine heart rate variability ⌐622

*Fig. 6*

```
                                                                        ┌─702
┌──────────────────────────────────────────────────────────────────────┐
│           Convert heart rate readings into heart rate reserve values   │
└──────────────────────────────────────────────────────────────────────┘
                                      │
                                      ▼                                 ┌─704
┌──────────────────────────────────────────────────────────────────────┐
│           Weight heart rate reserve values according to weighting scheme│
└──────────────────────────────────────────────────────────────────────┘
                                      │
                                      ▼                                 ┌─706
┌──────────────────────────────────────────────────────────────────────┐
│           Sum and normalize weighted time series of heart rate reserve values│
└──────────────────────────────────────────────────────────────────────┘
                                      │
                                      ▼                                 ┌─708
┌──────────────────────────────────────────────────────────────────────┐
│           Scale summed and normalized values to generate intensity score│
└──────────────────────────────────────────────────────────────────────┘
                                      │
                                      ▼                                 ┌─710
┌──────────────────────────────────────────────────────────────────────┐
│           Store intensity score on non-transitory computer-readable storage medium│
└──────────────────────────────────────────────────────────────────────┘
                                      │
                                      ▼                                 ┌─712
┌──────────────────────────────────────────────────────────────────────┐
│           Display intensity score on user interface rendered on display device│
└──────────────────────────────────────────────────────────────────────┘
```

*Fig. 7*

Fig. 8

Maximum All Out — 21, 20

Near Maximal — 19, 18

Very Hard Workout — 17, 16

Hard Workout — 15, 14

Moderate Workout — 13, 12

Light Workout — 11

Active — 10, 9

— 8

Light Activity — 7

— 6

— 5

— 4

No Activity — 3

— 2

— 1

Asleep — 0

19.0

Example

12.5

Moderate workout good intensity for a tapering workout, subject did not overcome his anaerobic threshold and will have little to no soreness tomorrow.

*Fig. 9*

Fig. 10

Heart Rate Variability

Resting Heart Rate

Sleep Quality

Recent Strain (Physical & Psychological)

Recovery Score

18%

Tactical athlete has not recovered from recent strain and may need rest

*Fig. 11A*

Heart Rate Variability

Resting Heart Rate

Sleep Quality

Recent Strain (Physical & Psychological)

Recovery Score

45%

Tactical athlete has average sleep and HRV readings indicating he has moderately recovered from recent strain. He is prepared for activity

*Fig. 11B*

Heart Rate Variability

Resting Heart Rate

Sleep Quality

Recent Strain (Physical & Psychological)

Recovery Score

83%

Tactical athlete is near fully recovered and showing signs of good fitness. He is well prepared for intense activity

*Fig. 11C*

*Fig. 12*

Fig. 13

Fig. 14

1500

```
┌─────────────────────────────────────┐
│  PROVIDE STRAP WITH SENSOR AND HEART │
│     RATE MONITORING SYSTEM           │
│              1502                    │
└─────────────────────────────────────┘
                  │
                  ▼
┌─────────────────────────────────────┐
│     DETECT SIGNAL FROM SENSOR        │
│              1504                    │
└─────────────────────────────────────┘
                  │
                  ▼
┌─────────────────────────────────────┐
│   DETERMINE CONDITION OF HEART RATE  │
│        MONITORING SYSTEM             │
│              1506                    │
└─────────────────────────────────────┘
                  │
                  ▼
┌─────────────────────────────────────┐
│    SELECT MODE BASED ON CONDITION    │
│              1508                    │
└─────────────────────────────────────┘
                  │
                  ▼
┌─────────────────────────────────────┐
│   STORE CONTINUOUS HEART RATE DATA   │
│              1510                    │
└─────────────────────────────────────┘
```

*Fig. 15*

1600

```
MONITOR DATA FROM
WEARABLE SYSTEM
1602
```

↓

```
DETECT EXERCISE ACTIVITY
1604
```

↓

```
GENERATE ASSESSMENT OF
EXERCISE ACTIVITY
1606
```

↓

```
DETECT RECOVERY STATE
1608
```

```
GENERATE ASSESSMENT OF
RECOVERY STATE
1610
```

↓

```
ANALYZE ASSESSMENTS
1612
```

↓

```
GENERATE RECOMMENDATION
1614
```

EP 4 165 658 B1

*Fig. 16*

*Fig. 17*

EP 4 165 658 B1

1800

ACQUIRE PHYSIOLOGICAL DATA
1802

GENERATE INDICATOR OF INFECTION
1804

COMMUNICATE INDICATOR TO USER
1806

*Fig. 18*

*Fig. 19*

EP 4 165 658 B1

*Fig. 20*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63037499
- US 2019167209 A1
- EP 3479758 A1
- WO 2017032873 A2

### Non-patent literature cited in the description

- **CAPODILUPO EMILY.** *The Importance of Respiratory Rate Tracking During The COVID-19 Pandemic,* 01 April 2020, www.whoop.com/us/en/thelocker/respiratory-rate-tracking-coronavirus
- **STOJANOVIC RADOVAN et al.** *A Headset Like Wearable Device to Track COVID-19 Symptoms*
- **CHARIOT K et al.** Interchangeability between heart rate and photoplethysmography variabilities during sympathetic stimulations. *Physiological Measurement.,* December 2009, vol. 30 (12), 1357-69, http://www.ncbi.nlm.nih.gov/pubmed/19864707
- **LU, S.** Can photoplethysmography variability serve as an alternative approach to obtain heart rate variability information?. *Journal of Clinical Monitoring and Computing,* February 2008, vol. 22 (1), 23-9, http://www.ncbi.nlm.nih.gov/pubmed/17987395
- **MILLER et al.** Analyzing changes in respiratory rate to predict the risk of COVID-19 infection. *PLoS ONE,* 10 December 2020, vol. 15 (12), e0243693, https://doi.org/10.1371/journal.pone.0243693
- **EBELL, M.H.** Predicting pneumonia in adults with respiratory illness. *Am. Fam. Physician,* 2007, vol. 76 (4), 560-2
- **PEDREGOSA F. et al.** Scikit-learn: Machine Learning in Python. *J. Mach. Learn. Res.,* 2011, vol. 12, 2825-30